(19) Europäisches Patentamt
European Patent Office
Office européen des brevets

(11) EP 3 598 898 A1

(12) EUROPEAN PATENT APPLICATION

(43) Date of publication:
**29.01.2020 Bulletin 2020/05**

(51) Int Cl.:
***A01N 63/00*** *(2020.01)* ***A01K 67/033*** *(2006.01)*

(21) Application number: **18186046.1**

(22) Date of filing: **27.07.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **e-nema Gesellschaft für Biotechnologie und biologischen Pflanzenschutz mbH**
**24223 Schwentinental (DE)**

(72) Inventors:
- **Ehlers, Prof. Dr. Ralf-Udo**
  **24223 Schwentinental (DE)**
- **Molina, Dr. Carlos**
  **24223 Schwentinental (DE)**
- **Vandenbossche, Dr. Bart**
  **24223 Schwentinental (DE)**
- **Strauch, Dr. Olaf**
  **24223 Schwentinental (DE)**

(74) Representative: **Lobemeier, Martin Landolf et al**
**c/o Ibmr. Patent- und Markenrecht**
**Holtenauer Strasse 57**
**24105 Kiel (DE)**

# (54) HETERORHABDITIS BACTERIOPHORA WITH ENHANCED SHELF LIFE

(57) Entomopathogenic nematode *Heterorhabditis bacteriophora* having an enhanced longevity, comprising a first locus comprising a single nucleotide polymorphism at position 75 of the nucleotide sequence SC00004647 as depicted in SEQ ID NO: 5, in which C is substituted by T; and/or a second locus comprising a single nucleotide polymorphism at position 54 of the nucleotide sequence SC00006203 as depicted in SEQ ID NO: 7, in which C is substituted by T.

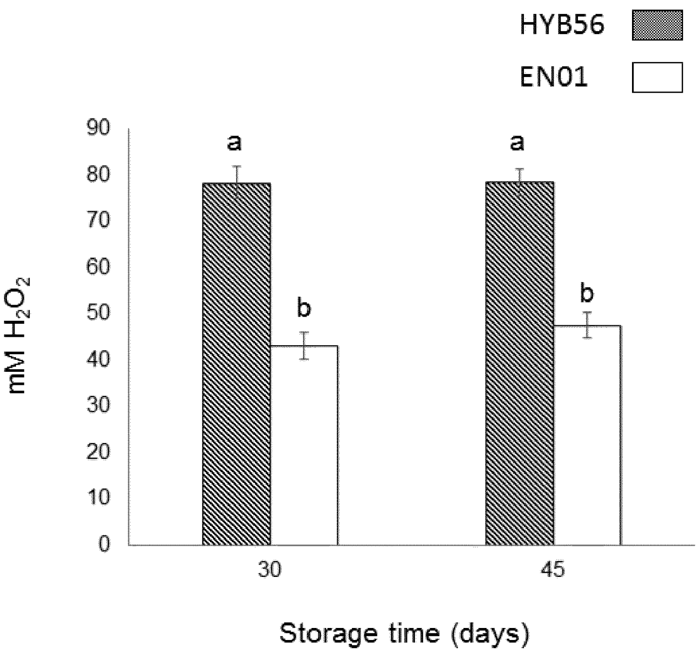


FIG.8

EP 3 598 898 A1

SEQUENCE LISTING

<110> e-nema Gesellschaft für Biotechnologie und biologischen Pflanzenschutz mbH

<120> Heterorhabditis bacteriophora with enhanced shelf-life

<130> E 5377

<160> 11

<170> BiSSAP 1.3.6

<210> 1
<211> 164
<212> DNA
<213> Heterorhabditis bacteriophora

<220>
<223> SC00003427

<400> 1
ttatcaagta aataaagttc gtctattttt attaagattt tctcactaaa gtgataagta 60

tgttgtagtt cttgattagt attaattaac agcgattaaa tgccagagag gcaataaacg 120

ctgtgtaaac ccacattaat ttagcttttt ctattcacag attc 164

<210> 2
<211> 178
<212> DNA
<213> Heterorhabditis bacteriophora

<220>
<223> SC00004141

<400> 2
tacatacttg cattaaatgg aacaaagtgc tcatcaatgt gcatttagta tttacatcta 60

tgtgtatgaa atgtgacatc tgtatattgt gcgaacttaa caaagaaaga cttattgagg 120

tcatttttat atacatggtg tccacgataa aaggacctat ttgacaagtt ttataact 178

<210> 3
<211> 164
<212> DNA
<213> Heterorhabditis bacteriophora

<220>
<223> SC00004554

<400> 3
aattaaaccg cagatgaccg agccaggggt gagtttttcg gtgcacttcg atgtgagttt 60

gaagactgcg agggatgtaa gtttactggt gagttttcct ttatattttt tttcagtact 120

ctcctgagcc gaggcgtttg cctcagtgct cttttcccac ctcc 164

<210> 4
<211> 180
<212> DNA
<213> Heterorhabditis bacteriophora

<220>
<223> SC00004634

<400> 4
atcttttagg aagtacaaaa gatgtataat ttatttacta gtaataattc gccacgttct 60

tctactatcc atgttgatgt tgtcactatg tttaagcact tgataggtat atggatacac 120

atctgagatt tcgtgtcatt tactttaccc cggttacttt tcgggctatt ttataccctt 180

<210> 5
<211> 164
<212> DNA
<213> Heterorhabditis bacteriophora

<220>
<223> SC00004647

<400> 5
aacttagtaa taaaattcgt aaaaattatt ttatgcttac attcactcct atggacttct 60

acatagaagg cttccgatga gcggggaata agccctcgct gtccagtggc aatattcatc 120

gcatccagtg aacaatccct ttaatatgtg aaacttaaag ttgg 164

<210> 6
<211> 232
<212> DNA

<213> Heterorhabditis bacteriophora

<220>
<223> SC00005330

<400> 6
ctattactac tactattact attatcaagt tgagttaaat taataaaggt gaaaatattg 60

tggcattatt tttgacatgc ctgtggtttg aatcactgct tttttttatc atgattttta 120

ttctagaatg gtaccaaatt gtatagtaaa ggcgaagaac gaaaggaagc gagtatacgt 180

cggtgaagaa ttatgtgaat gtgctgatcg aagcagccta ttcttcgtat ta 232

<210> 7
<211> 164
<212> DNA
<213> Heterorhabditis bacteriophora

<220>
<223> SC00006203

<400> 7
tcaccgaaat attgtggtag aagttagcgt gagaagttgg actcatatta gtgctattca 60

tcgaatggac atgggaaaac agttactcag ataactgttc ctttgccctg tgaataaggg 120

cagatttaat cttacggtta ctggtcttca tggctgaaca actt 164

<210> 8
<211> 164
<212> DNA
<213> Heterorhabditis bacteriophora

<220>
<223> SC00010093

<400> 8
gtggcgagaa gaaagaataa gtattatttg aaagatcaat atccattaat atgagtgaac 60

aattgaatag gacaatagtt aaatgataga aggtttaact caatggttaa gtttaaaaag 120

ataagggaac tacttcagac aggtcttcgg cgcacggaat cggc 164

<210> 9
<211> 164
<212> DNA
<213> Heterorhabditis bacteriophora

<220>
<223> SC00012917

<400> 9
ttgttccatt gttcaaaaac attgtaatac tgtcaactat tgcttggaac atgttctaga 60

ataatggttc attggccatt tgccgtcatt agtaatgtta aaatagtttt aatctgtagt 120

ggatttgtgg cggacgcagt ggttgatgca tcagaattgt tcca 164

<210> 10
<211> 318
<212> DNA
<213> Heterorhabditis bacteriophora

<220>
<223> EN-Hb_oxid-11688

<400> 10
gcttcatggt tttagccata caatcgatga tgccattata ggcattcatg gtttgtagtc 60

tagccttgac tgtgtccagc ggatgtccaa cgagaaggcc tgctcctcct aacatattaa 120

aggtcatagc cgatctcgcc cgccccattt tctcagcgta aataatcaat cgaactacga 180

gaggtcaacc aaacggctgg actgcttatt gacagttttg ctgttagcgt tctcgtattt 240

tatatttgca cctcattata ttttagtttg tctaattaaa tatatgaact aattgataaa 300

taaataggtt cttacttc 318

<210> 11
<211> 376
<212> DNA
<213> Hctcrorhabditis bactcriophora

<220>
<223> EN-Hb_oxid-26008

<400> 11

caaaaattcc gatcaacata ctttatacat tttatcgtta tgaagtcatt tattcattga 60

ctgagaaaaa tataagtgaa gagccactaa ttaatcgata tataagtagc tacaagattg 120

atttttaata ctattgtaaa taataattag ttaaatgcat tgtagcaaat taaaaactaa 180

tgatatctaa gaaaatcccg gaagaaaagg atacgaaacg gtcatctaac aacgctataa 240

taattatgca gttttaattt tcttgctatt aaaaaatcgt aacaataaca ttgatacata 300

tatatcgatt aattagtggc tcttcactta tatttttctc agtcaatgaa taaatgactt 360

cataacgata aaatgt 376

SEQUENCE LISTING

<110> e-nema Gesellschaft für Biotechnologie und biologischen Pflanzenschutz mbH

<120> Heterorhabditis bacteriophora with enhanced shelf-life

<130> E 5377

<160> 11

<170> BiSSAP 1.3.6

<210> 1
<211> 164
<212> DNA
<213> Heterorhabditis bacteriophora

<220>
<223> SC00003427

<400> 1
ttatcaagta aataaagttc gtctattttt attaagattt tctcactaaa gtgataagta 60

tgttgtagtt cttgattagt attaattaac agcgattaaa tgccagagag gcaataaacg 120

ctgtgtaaac ccacattaat ttagcttttt ctattcacag attc 164

<210> 2
<211> 178
<212> DNA
<213> Heterorhabditis bacteriophora

<220>
<223> SC00004141

<400> 2
tacatacttg cattaaatgg aacaaagtgc tcatcaatgt gcatttagta tttacatcta 60

tgtgtatgaa atgtgacatc tgtatattgt gcgaacttaa caaagaaaga cttattgagg 120

tcatttttat atacatggtg tccacgataa aaggacctat ttgacaagtt ttataact 178

<210> 3
<211> 164
<212> DNA
<213> Heterorhabditis bacteriophora

<220>
<223> SC00004554

<400> 3
aattaaaccg cagatgaccg agccaggggt gagtttttcg gtgcacttcg atgtgagttt 60

gaagactgcg agggatgtaa gtttactggt gagttttcct ttatattttt tttcagtact 120

ctcctgagcc gaggcgtttg cctcagtgct cttttcccac ctcc 164

<210> 4
<211> 180
<212> DNA
<213> Heterorhabditis bacteriophora

<220>
<223> SC00004634

<400> 4
atcttttagg aagtacaaaa gatgtataat ttatttacta gtaataattc gccacgttct 60

tctactatcc atgttgatgt tgtcactatg tttaagcact tgataggtat atggatacac 120

atctgagatt tcgtgtcatt tactttaccc cggttacttt tcgggctatt ttataccctt 180

<210> 5
<211> 164
<212> DNA
<213> Heterorhabditis bacteriophora

<220>
<223> SC00004647

<400> 5
aacttagtaa taaaattcgt aaaaattatt ttatgcttac attcactcct atggacttct 60

acatagaagg cttccgatga gcggggaata agccctcgct gtccagtggc aatattcatc 120

gcatccagtg aacaatccct ttaatatgtg aaacttaaag ttgg 164

<210> 6
<211> 232
<212> DNA
<213> Heterorhabditis bacteriophora

<220>
<223> SC00005330

<400> 6
ctattactac tactattact attatcaagt tgagttaaat taataaaggt gaaaatattg 60

tggcattatt tttgacatgc ctgtggtttg aatcactgct ttttttatc atgattttta 120

ttctagaatg gtaccaaatt gtatagtaaa ggcgaagaac gaaaggaagc gagtatacgt 180

cggtgaagaa ttatgtgaat gtgctgatcg aagcagccta ttcttcgtat ta 232

<210> 7
<211> 164
<212> DNA
<213> Heterorhabditis bacteriophora

<220>
<223> SC00006203

<400> 7
tcaccgaaat attgtggtag aagttagcgt gagaagttgg actcatatta gtgctattca 60

tcgaatggac atgggaaaac agttactcag ataactgttc ctttgccctg tgaataaggg 120

cagatttaat cttacggtta ctggtcttca tggctgaaca actt 164

<210> 8
<211> 164
<212> DNA
<213> Heterorhabditis bacteriophora

<220>
<223> SC00010093

<400> 8
gtggcgagaa gaaagaataa gtattatttg aaagatcaat atccattaat atgagtgaac 60

aattgaatag gacaatagtt aaatgataga aggtttaact caatggttaa gtttaaaaag 120

ataagggaac tacttcagac aggtcttcgg cgcacggaat cggc 164

<210> 9
<211> 164
<212> DNA
<213> Heterorhabditis bacteriophora

<220>
<223> SC00012917

<400> 9
ttgttccatt gttcaaaaac attgtaatac tgtcaactat tgcttggaac atgttctaga 60

ataatggttc attggccatt tgccgtcatt agtaatgtta aaatagtttt aatctgtagt 120

ggatttgtgg cggacgcagt ggttgatgca tcagaattgt tcca 164

<210> 10
<211> 318
<212> DNA
<213> Heterorhabditis bacteriophora

<220>
<223> EN-Hb_oxid-11688

<400> 10
gcttcatggt tttagccata caatcgatga tgccattata ggcattcatg gtttgtagtc 60

tagccttgac tgtgtccagc ggatgtccaa cgagaaggcc tgctcctcct aacatattaa 120

aggtcatagc cgatctcgcc cgccccattt tctcagcgta aataatcaat cgaactacga 180

gaggtcaacc aaacggctgg actgcttatt gacagttttg ctgttagcgt tctcgtattt 240

tatatttgca cctcattata ttttagtttg tctaattaaa tatatgaact aattgataaa 300

taaataggtt cttacttc 318

```
<210> 11
<211> 376
<212> DNA
<213> Heterorhabditis bacteriophora

<220>
<223> EN-Hb_oxid-26008

<400> 11
caaaaattcc gatcaacata ctttatacat tttatcgtta tgaagtcatt tattcattga      60

ctgagaaaaa tataagtgaa gagccactaa ttaatcgata tataagtagc tacaagattg     120

atttttaata ctattgtaaa taataattag ttaaatgcat tgtagcaaat taaaaactaa     180

tgatatctaa gaaaatcccg gaagaaaagg atacgaaacg gtcatctaac aacgctataa     240

taattatgca gttttaattt tcttgctatt aaaaaatcgt aacaataaca ttgatacata     300

tatatcgatt aattagtggc tcttcactta tatttttctc agtcaatgaa taaatgactt     360

cataacgata aaatgt                                                     376
```

## Description

The invention relates to an entomopathogenic nematode as a biocontrol agent with enhanced shelf life and virulence. Particularly, the invention relates to *Heterorhabditis bacteriophora* (Poinar 1975) as a biocontrol agent having an enhanced shelf life and virulence.

*Heterorhabditis bacteriophora* (Poinar) is an entomopathogenic nematode (EPN) species with a broad spectrum in infectivity. The market for biocontrol agents (BCA) is nowadays under constant growth and nematodes are entering from niche markets into large scale field cultures.

The infective dauer juvenile (DJ) is the only free-living stage of this entomopathogenic nematode, being the active form as biocontrol agent against insect pests. These dauer juveniles are shear stress tolerant and can be easily applied using conventional spraying technology. Industrially, *H. bacteriophora* DJ are produced in bioreactors (monoxenic liquid culture) together with their symbiotic bacterium *Photorhabdus luminescens.* Infective DJs can be stored in liquid suspension before they are formulated in powder, transported and applied on fields or in greenhouses.

During storage, the DJ mortality is gradually increasing along with the loss of virulence. As the nematode market grows, bigger production batches are required. This increase in demand and production calls for extended storage times (shelf life) for *H. bacteriophora* based biocontrol products.

Further, the use of nematodes in field crops is a severe physiologic challenge for these nematodes. One example is the control of the Western Corn Rootworm (*Diabrotica virgifera virgifera*) in maize plantations. In Europe, DJs are applied at the sowing time (around March/April) and have to remain viable in the soil until the target insect larvae emerge from the egg (around May). During this time, the DJ are exposed to stresses like drought and high temperatures also resulting in oxidative stress and reduction of the nematode population. Thus, the improvement of the survival (DJ-longevity) during storage and after application (field persistence) is a major task for the use of *H. bacteriophora* as a biocontrol agent.

Hence, it is a first object of the present invention to provide a genetically distinct *Heterorhabditis bacteriophora* organism having an improved shelf life and virulence. Particularly, it is a second object of the present invention to provide a genetically distinct *Heterorhabditis bacteriophora* organism having a prolonged shelf life and increased virulence as a source of a biocontrol agent against insect pests. Specifically, it is further object to provide a genetically distinct *Heterorhabditis bacteriophora* organism having an improved shelf life and virulence as a biocontrol agent against the Western Corn Rootworm *(Diabrotica virgifera virgifera*).

The invention is based on the finding of a strong correlation between oxidative stress tolerance and DJ-longevity in entomopathogenic nematode (EPN) species like *Heterorhabditis bacteriophora.* Also, genetic crosses and phenotypic selections were done in natural strains leading to an increase in survival time. A large body of sequence data comprising more than 55.000 transcripts and more than 1.100 single nucleotide polymorphisms (SNPs) from contrasting materials in virulence- and DJ-longevity have been generated and deeply analyzed. Thereafter, relevant information has been transferred into genotyping molecular markers to test the genotype-phenotype correlation. As outcome, a set of PCR-based molecular markers that show association with EPN beneficial traits (DJ-longevity and virulence) have been identified. A series of *H. bacteriophora* strains, inbred lines and mutants has been also resulting from the virulence and DJ-longevity characterization. Among them, the HYB56 and the resulting inbred line HYB56-IL73 have shown the most robust performance results in the control of *Diabrotica v. virgifera,* in contrast to the current commercial *H. bacteriophora* strain EN01 and also compared to the standard chemical control measures.

Accordingly, there is claimed an entomopathogenic nematode *Heterorhabditis bacteriophora* having an enhanced longevity, comprising a first locus, comprising a single nucleotide polymorphism at position 75 of the nucleotide sequence SC00004647 as depicted in SEQ ID NO: 5, in which C is substituted by T; and/or a second locus comprising a single nucleotide polymorphism at position 54 of the nucleotide sequence SC00006203 as depicted in SEQ ID NO: 7, in which C is substituted by T.

Preferably, the entomopathogenic nematode further is having a third locus comprising a single nucleotide polymorphism at position 66 of the nucleotide sequence SC00003427 as depicted in SEQ ID NO: 1, in which T is substituted by G; and/or a fourth locus comprising a single nucleotide polymorphism at position 76 of the nucleotide sequence SC00004141 as depicted in SEQ ID NO: 2, in which A is substituted by T; and/or a fifth locus comprising a single nucleotide polymorphism at position 86 of the nucleotide sequence SC00004634 as depicted in SEQ ID NO: 4, in which C is substituted by T; and/or a sixth locus comprising a single nucleotide polymorphism at position 98 of the nucleotide sequence SC00005330 as depicted in SEQ ID NO: 6, in which G is substituted by A; and/or a seventh locus comprising a single nucleotide polymorphism at position 77 of the nucleotide sequence SC00012917 as depicted in SEQ ID NO: 9, in which C is substituted by G; and/or an eighth locus comprising a single nucleotide polymorphism at position 200 of the nucleotide sequence EN-Hb_oxid-11688 as depicted in SEQ ID NO: 10, in which G is substituted by C; and/or a ninth locus comprising a single nucleotide polymorphism at position 176 of the nucleotide sequence EN-Hb_oxid-26008 as depicted in SEQ ID NO: 11, in which A is substituted by G.

As a further preferred embodiment of the invention the entomopathogenic nematode is having a tenth locus conferring an enhanced virulence, comprising a single nucleotide polymorphism at position 73 of the nucleotide sequence SC00004554 as depicted in SEQ ID NO: 3, in which G is substituted by A.

Alternatively or additionally, the entomopathogenic nematode is having an eleventh locus conferring an enhanced virulence, comprising a single nucleotide polymorphism at position 111 of the nucleotide sequence SC00010093 as depicted in SEQ ID NO: 8, in which G is substituted by A.

Moreover, there is claimed a method of identifying at least one individual of entomopathogenic nematode *Heterorhabditis bacteriophora* associated with enhanced longevity, by determining a first locus comprising a single nucleotide polymorphism at position 75 of the nucleotide sequence SC00004647 as depicted in SEQ ID NO: 5, in which C is substituted by T; and/or a second locus comprising a single nucleotide polymorphism at position 54 of the nucleotide sequence SC00006203 as depicted in SEQ ID NO: 7, in which C is substituted by T.

Preferably the method comprises determining a third locus comprising a single nucleotide polymorphism at position 66 of the nucleotide sequence SC00003427 as depicted in SEQ ID NO: 1, in which T is substituted by G; and/or a fourth locus comprising a single nucleotide polymorphism at position 76 of the nucleotide sequence SC00004141 as depicted in SEQ ID NO: 2, in which A is substituted by T; and/or a fifth locus comprising a single nucleotide polymorphism at position 86 of the nucleotide sequence SC00004634 as depicted in SEQ ID NO: 4, in which C is substituted by T; and/or a sixth locus comprising a single nucleotide polymorphism at position 98 of the nucleotide sequence SC00005330 as depicted in SEQ ID NO: 6, in which G is substituted by A; and/or a seventh locus comprising a single nucleotide polymorphism at position 77 of the nucleotide sequence SC00012917 as depicted in SEQ ID NO: 9, in which C is substituted by G; and/or an eighth locus comprising a single nucleotide polymorphism at position 200 of the nucleotide sequence EN-Hb_oxid-11688 as depicted in SEQ ID NO: 10, in which G is substituted by C; and/or a ninth locus comprising a single nucleotide polymorphism at position 176 of the nucleotide sequence EN-Hb_oxid-26008 as depicted in SEQ ID NO: 11, in which A is substituted by G.

Also, there is claimed a method of identifying at least one individual of entomopathogenic nematode *Heterorhabditis bacteriophora* associated with enhanced virulence, by determining a tenth locus conferring an enhanced virulence, comprising a single nucleotide polymorphism at position 73 of the nucleotide sequence SC00004554 as depicted in SEQ ID NO: 3, in which G is substituted by A or by determining an eleventh locus conferring an enhanced virulence, comprising a single nucleotide polymorphism at position 111 of the nucleotide sequence SC00010093 as depicted in SEQ ID NO: 8, in which G is substituted by A.

Based on the above a biological control agent is provided comprising the entomopathogenic nematode having at least one of the above identified loci and/or which has been determined by the aforementioned methods.

As a preferred embodiment the biological control agent is provided comprising at least one agriculturally acceptable carrier.

Finally, according to the invention, the use of the entomopathogenic nematode having the features of the invention against true weevils (*Curculionidae*), scarabs (*Scarabaeidae*) or leaf beetles (*Chrysomelidae*) is provided, wherein the use against the Western Corn Rootworm (*Diabrotica virgifera virgifera*) is particularly preferred.

## GENERAL REMARKS

*Heterorhabditis bacteriophora* Poinar is an effective biological control agent (BCA) against insect pests in economically important crops (Grewal et al., 2005a). This species has a symbiotic association with the bacterium *Photorhabdus luminescens,* which is carried by the nematodes in the free-living, developmentally arrested dauer juvenile (DJ) stage. *Heterorhabditis bacteriophora* is used commercially mainly for the control of several curculionid weevil larvae in soft fruit and ornamentals (Long et al., 2000) and against white grubs (Guo et al., 2013). This EPN species has also large potential as BCA against the invasive maize pest Western Corn Rootworm *Diabrotica virgifera virgifera* (Toepfer et al., 2005).

Despite several advantages offered by this species, its use in larger scale agriculture is restricted by a limited shelf life and high sensitivity to environmental stress conditions (Strauch et al., 2004; Mukuka et al., 2010a and b). DJ of *H. bacteriophora* are industrially produced in monoxenic liquid culture in bioreactors and are stored in refrigerated liquid suspension at high densities for maximum 6 weeks after production (Ehlers, 2001). For transport and storage to the end-users, DJ need to be formulated under moderate desiccation in a powder (Grewal and Peters, 2005). Additional to the stress to which EPN are exposed during production and transport, post-application stress factors such as UV-radiation, high temperatures, drought and oxidative stress reduce the DJ-survival (Grewal et al., 2002; Strauch et al., 2004; Ehlers et al., 2005; Mukuka et al., 2010a; Sumaya et. al, 2017). Thus, improvement of DJ-longevity and virulence are priority tasks for *H. bacteriophora* as BCA.

Classical genetics have been applied in beneficial traits-improvement using the phenotypic and genetic natural variability as starting points. Crossbreeding and successive genetic selection, for instance, have evidenced a significant heat and desiccation stress-tolerance improvement in *H. bacteriophora.* For instance, Ehlers et al. (2005) increased the mean tolerated temperature of a *H. bacteriophora* hybrid strain to 39.2°C for 2 hours. Subsequently, Mukuka et al.

(2010a, b) screened 60 *H. bacteriophora* strains from different geographical origin and reported an increase to 44.0 °C for 2 hours after eleven selection steps. Other traits related to EPNs such as virulence appear to have more complex genetic backgrounds due to influence of several factors such as host-finding ability, persistence, and infective-related aspects. Up to date, no correlation between stress-tolerance and virulence in EPNs has been found (Shapiro-Ilan et al., 2015).

Understanding the physiological and molecular mechanisms behind the DJ-longevity and virulence in *H. bacteriophora* should open doors in the search for molecular markers associated to these traits. Molecular markers can be applied in breeding programs where they can be used as trait predictors. Sequence data availability on this species for the design of molecular markers has only been gradually increasing during the past ten years. For instance, Bai et al. (2009) reported on the availability of 168 microsatellite loci from 157 distinct *H. bacteriophora* Expressed Sequence Tag (EST) sequences, which were generated by cDNA libraries sequencing. In a parallel work, Regeai et al. (2009) reported on the availability of 24 intron-derived markers in housekeeping and structural genes of *H. bacteriophora.* Subsequently, a primary draft sequence of the *H. bacteriophora* genome was released in 2013 by Bai and co-authors. Only until recently, Vadnal et al (2017) reported on the first published RNA-seq analysis of *H. bacteriophora* DJs, using the current genome draft as reference sequence.

In the framework of the EU Project BIOCOMES, three deep RNA-seq analyses were carried out yielding more than 55.000 de novo assembled transcripts. Additionally, SNPs were sequenced in genomic DNA using the Genotyping by Sequencing (GBS) method, yielding more than 1200 polymorphic SNPs. Thereafter, SNP information was used to construct a low-resolution linkage map, which was combined with phenotypic information to carry out a DJ-longevity QTL analysis. As outcome, correlation analysis between genotype and phenotype data yielded twelve PCR-based SNP markers that can be used as DJ-longevity predictors. Additionally, two of these markers show also high association with DJ-virulence.

To evaluate the versatility of the wild type, hybrid and inbred line strains identified and generated, we assessed their performance in the control of the Western Corn Rootworm or WCR (*Diabrotica virgifera virgifera* LeConte; Coleoptera: Chrysomelidae) in maize plantations. Supposed to have originated and evolved together with maize in Central America (Krysan & Smith, 1987), the WCR turned into a key pest when maize expanded to North America (Toepfer et al., 2005, 2010). Followed by several introductions from the USA, WCR has rapidly spread in central Europe, becoming a significant threat to maize production principally in some countries such as Austria, Hungary, Serbia, Romania and Italy (Kiss et al., 2005; Toepfer et al., 2010). Concerning biological control, *H. bacteriophora* has been previously appointed as a potential BCA for *D. v. virgifera* (Toepfer et al., 2005). However, post-application environmental stresses such as desiccation, change in temperatures and time-lapse without host larvae still substantially reduce the DJ-efficacy of this EPN. Thus, H. *bacteriophora* strains and inbred lines with enhanced stress-tolerance, longevity and virulence have a great potential for the biological control of this pest.

Two approaches were followed to achieve improved beneficial traits in *H. bacteriophora* as BCA (Figure 1): i) identification of strains and lines that already combine high virulence and DJ-longevity, and ii) combination of properties by genetic crossing and selection. In this context, the wild type (WT) strains have been identified, combining high-virulence and DJ-longevity. These WT strains were evaluated individually and were used to generate hybrid pools by crossing the WT strains. The hybrid pool HYB56 derived from the crosses and inbred line HYB56-IL73 derived from the hybrid by inbreeding have also shown improved performance. Extensive virulence and DJ-longevity characterization starting from laboratory assays up to field trials in central Europe confirm the phenotypic improvement as BCA against *D. v. virgifera.*

## BRIEF DESCRIPTION OF THE FIGURES

Main experimental steps and milestones for the generation and evaluation of natural strains and hybrids are depicted in the accompanying drawings, wherein:

Fig. 1 shows a flow chart presenting the main experimental steps carried out towards the testing and selection of *Heterorhabditis bacteriophora* materials with improved performance.

Fig. 2 shows the mean $LD_{50}$ of DJs per *Tenebrio molitor* larva of a set of *Heterorhabditis bacteriophora* WT strains and inbred lines. Mortality was assessed after 7 days of exposure to different numbers of DJs at 25°C. Error bars: SD of four replicates. Different letters on the error bars indicate significant differences (Conover-Iman test with Bonferroni correction at $P \leq 0,0001$)

Fig. 3 shows the Mean Time Survived by 50% ($MTS_{50}$) of two DJ-populations from the contrasting lines DE6-IL4 (high survival) and DE2-IL1 (low survival). Both inbred lines derived from 8 cycles of self-fertilization. Error bars: SD of three independent trials. Different letters denote significant differences (Tukey's HSD test at $P \leq$

0.05).

Fig. 4 shows the Mean Time Survived by 50% ($MTS_{50}$) of recombinant inbred lines (D2D6-RILs) originating from the parental hybrid D2D6 and parental lines D2 and D6 under oxidative stress conditions. DJs of each line were treated with 70mM $H_2O_2$ at 25 °C in three randomized replicates and surviving nematodes were counted along 30 days. Arrows depict the two contrasting parents, DE6-IL4 (black) and DE2-IL1 (white). Error bars: SD. Letters at the right indicate significant differences among lines (Tukey's HSD test at $P \leq 0.05$).

Fig. 5 shows linkage groups obtained from polymorphic SNPs analyzed in 28 RILs from the D2D6 RILs along their parental lines. Five major linkage groups were determined comprising ~550 cM. Seven QTL were identified using data from one desiccation (white bar) and two oxidative stress trials (black bars), independently. Six of the seven QTL were found mainly in three linkage group regions. GBS clusters selected for design of PCR markers are denoted with a star.

Fig. 6 shows the mean $LD_{50}$ of the *Heterorhabditis bacteriophora* commercial strain EN01, HYB56 and HYB56-IL73 for last instar insect host larvae *Tenebrio molitor* (ruled dark bars) and last instar L3 larvae *Diabrotica virgifera virgifera* (white bars). $LD_{50}$ was assessed after seven days exposure of the insect larvae to different DJ numbers at 25 °C. Error bars: SEM of ten replicates for (*T. molitor*) and four replicates (*D. v. virgifera*). Different letters (uppercase for *T. molitor,* lowercase for *D. v. virgifera*) indicate significant differences (Tukey's HSD test at $P \leq 0.05$).

Fig. 7 shows the persistence (longevity) of *Heterorhabditis bacteriophora* commercial strain EN01, hybrid strain HYB56 and inbred line HYB56-IL73 in a laboratory assay. DJs of each strain were applied to potted maize plants and insects (*Diabrotica v. virgifera* (A) and *Tenebrio molitor* (B) were added 21 days later. Ten insects were used per pot to check for DJ persistence. Data represent percent insect mortality. Error bars: SD

Fig. 8 shows the $H_2O_2$ concentration (in mM) at which 50% of the nematode population (ENOl and HYB56) survived a 12 day treatment after storage at 6°C in a powder formulation for 30 or 45 days. Oxydative stress tolerance is correlated with DJ longevity. Error bars: SD from three replicates. Different letters on the bars denote significant differences within one time point (Tukey's Test, $P \leq 0.05$).

Fig. 9 shows the mean lethal dose ($LD_{50}$) of DJ populations (ENOl and HYB56) extracted from a powder formulation after storage of 30, 45 and 63 days at 6°C. Error bars: SD of three replicates. Different letters on bars denote significant differences within each time point (Tukey's test, $P \leq 0.05$).

Fig. 10 shows the efficacy of *Heterorhabditis bacteriophora* commercial strain ENOl and hybrid strain HYB56 (each $2 \times 10^9$ DJs $ha^{-1}$) against the untreated control and the chemical standard (Cypermethrin granules 12 kg $ha^{-1}$) in two field trials at two different locations (A) and (B) in Styria, Austria, with a natural population of *D. v. virgifera.* Nematodes and insecticide were applied during sowing in April into the furrow with the sowing machine (Monosem 4) equipped with injectors for nematode application. Nematodes were applied with 200 ltr. water $ha^{-1}$. Number of emerging adults was assessed with photoeclectors covering 5 plants 100 days (end of July) after application.

Fig. 11 shows the results on plant lodging of the same field trial as described in Fig. 10. Results indicate percentage of plants with damage (lodging) caused by feeding of rootworm larvae on the roots. Plants inclined < 30° with gooseneck symptoms (black), with > 30° inclination (grey) and lodging plants, which cannot be harvested (white). *Heterorhabditis bacteriophora* commercial strain ENOl and hybrid strain HYB56 (each $2 \times 10^9$ DJs $ha^{-1}$) were compared against the untreated control and the chemical standard (Cypermethrin granules 12 kg $ha^{-1}$) in two field trials at two different locations (A) and (B) in Styria, Austria, with a natural population of *D. v. virgifera.* Nematodes and insecticide were applied during sowing in April into the furrow with the sowing machine (Monosem 4) equipped with injectors for nematode application. Nematodes were applied with 200 ltr. water $ha^{-1}$.

Fig. 12 shows the persistence (longevity) of *Heterorhabditis bacteriophora* commercial strain EN01 and hybrid strain HYB56 against the untreated control in the field trial in Austrian (A) and Hungarian (B) locations. Sixty days post-DJ-application of one and two billion DJs per ha (1 and $2 \times 10^9$ DJs $ha^{-1}$) five plastic tubes per treatment containing 10 *Tenebrio molitor* larvae per tube were deposited at 10 cm depth in the soil and collected 10 days later to assess insect mortality. Error bars: SD

Fig. 13 shows the efficacy (Abbott corrected reduction of adult *Diabrotica v. vigifera* in %) of *Heterorhabditis bacteriophora* commercial strain EN01 and hybrid strain HYB56 (each $2 \times 10^9$ DJs $ha^{-1}$) and the chemical standard (Cypermethrin granules 12 kg $ha^{-1}$) in a plot field trial in Hungary. Nematodes and insecticide were applied during sowing in April into the furrow with the sowing machine equipped with injectors for nematode application. Nematodes were applied with 200 ltr. water $ha^{-1}$. In the beginning of May, plants were infested with 500 viable and ready-to-hatch *Diabrotica v. virgifera* eggs per plant per each of the treatments when the plants were at 1 to 2-leaf stage. All treatments were compared against the untreated control plots. For recording adult emergence, six consecutive maize plants were cut to a height of about 1000 cm and covered with gauze cages (inner size, 1.: 1,25 cm, w.: 40 cm, h.:1,50 cm, covering 6 maize plants). The sample size was 4 cages á 6 plants, thus totally 24 plants per treatment per field. Error bars: SD.

Fig. 14 shows the Abbott corrected reduction (in %) of the plant root damage (IOWA scale) assessed during the field trail described in Fig. 13. In April *Heterorhabditis bacteriophora* commercial strain EN01 and hybrid strain HYB56 ($2 \times 10^9$ DJs $ha^{-1}$) and the chemical standard (Cypermethrin granules 12 kg ha-1) were applied in a plot field trial in Hungary. Nematodes and insecticide were applied during sowing into the furrow with the sowing machine equipped with injectors for nematode application. Nematodes were applied with 200 ltr. water ha-1. In the beginning of May, plants were infested with 500 viable and ready-to-hatch *Diabrotica v. virgifera* eggs per plant per each of the treatments when the plants were at 1 to 2-leaf stage. All treatments were compared against the untreated control plots. Error bars: SD

## DETAILED DESCRIPTION OF THE INVENTION

### Nematodes growth in monoxenic cultures

Starting from a collection of *H. bacteriophora* strains, hybrid, and inbred lines that have been worked on in the lab in previous research, the isolates were propagated in Nematode Gelrite Media (NGG; gelrite 3.0 $gl^{-1}$, peptone 2.50 $gl^{-1}$, NaCl 51 mM, $CaCl_2$*$H_2O$ 1 mM, $MgSO_4$*$7H_2O$ 1 mM, $KH_2PO_4$ 1 mM, cholesterol 12 $\mu$M) pre-coated with pre-cultured *Photorhabdus luminescens* at a density of $2 \times 10^9$ cells $ml^{-1}$ in a semi-solid NGG matrix (NGG, 1.5$gl^{-1}$ gelrite). After DJ-recovery and completion of one life-cycle (~7 days), mature hermaphrodites (in stage of *endotokia matricida*, according to Johnigk & Ehlers (1999)) were washed-off from the NGG plates and re-suspended in Ringer's solution (NaCl 9 $gl^{-1}$, KCl 4.42 $gl^{-1}$, $CaCl_2 \times 2H_2O$ 0.37 $gl^{-1}$, $NaHCO_3$ 0.2 $gl^{-1}$) until the majority of DJ were harvested. Thereafter, DJs were cleaned via cotton trap and vacuum-filtering with a 10 $\mu$m sieve. Clean DJs were stored in culture flasks in Ringer's solution until used for characterization.

### Oxidative stress assays to predict DJ-longevity in strains and inbred lines

Dauer juveniles of *H. bacteriophora* wild type strains were subjected to oxidative stress assays according to Sumaya et al. (2017). DJ-populations from each line (~1,000 individuals) were disposed in 24-cell well plates in a final volume of 400 $\mu$l of Ringer's solution in three randomized technical replicates. Thereafter, 15 $\mu$l of 1.94 M $H_2O_2$ were added to each cell-well to obtain a final $H_2O_2$ concentration of 70 mM. DJ-mortality over time was assessed by periodically surveying (~every second day) 20 $\mu$l aliquots for dead and alive individuals in each replicate. The percentage of DJ-mortality was used to determine the mean time survived by 50% of the population ($MTS_{50}$) of the DJ-population for each line. The $MTS_{50}$ was determined from a fitted cumulative normal distribution by using the Probit analysis of the XLSTAT (https://www.xlstat.com/de/) software. Oxidative stress assays were repeated starting from different DJ growth batches.

### Assessment of virulence

Mealworms (*Tenebrio molitor*) were used as hosts for the virulence characterization of *H. bacteriophora* WT materials. *T. molitor* larvae were obtained from Futterinsektenfarm Schulz (Eschach-Holzhausen, Germany). For laboratory virulence bioassays, 40 *T. molitor* larvae were placed into Petri dishes (150 mm) filled with 150 g of sand adjusted to 8.5 % water content. Nematode suspension volumes of 1.0 ml (Ringer's solution) from each strain and line containing 80, 200, 400, 800 and 2000 DJs $ml^{-1}$ were inoculated in the middle of the Petri dishes and incubated at 25°C for 7 days. Infection by DJs was checked by recording the bioluminescence with the luminometer LUMAT LB 9501 (Berthold GmbH, Germany), resulting from growth of the nematode-symbiotic bacteria in the cadavers. The mean mortality by nematode infection of *T. molitor* was compared among all strains. Mortality data were used to calculate the lethal dose of DJs required to kill 50% of the insect larvae per assay ($LD_{50}$). The $LD_{50}$ was calculated by fitting the observed data to saturated curves, which were compared with the saturation curve through minimizing the chi-square ($chi^2$) fitting to the nearest value to zero. The insect mortality was calculated through the formula:

$$\text{Mortality} = a\,(1\text{-ln}\,(\text{-}bx))+c$$

Where:

a = total number of insects
b = slope of the fitted model
x = number of DJs per insect
c = control mortality

The data were compared with the saturation curve through minimizing the chi-square ($Chi^2$) fitting to a value nearest to zero (0). The values obtained were analyzed for normality with the Shapiro-Wilk test at $P \leq 0.05$. In case of non-normal-distribution, log transformation was used. For normal distributed data ANOVA and Tukey's test for multiple comparisons were done. For non-normal distributed data the Kruskal-Wallis test with post hoc Conover-Iman test for multiple comparisons were used. The Bonferroni test was performed for correction of significant differences. Standard deviation of $LD_{50}$ was calculated according to the formula:

$$SD= \sqrt{}(\text{infected rate} \times \text{non-infected} \times \text{total insects}) \div (\text{total insects})$$

**Variability in DJ-longevity and virulence among *H. bacteriophora* strains**

Bioassays to assess the mean time survived by 50% of a DJ population at commercial conditions of 4-8°C can last for several months. Therefore, longevity was tested at 25°C. To further reduce the time scale of such experiments, DJs were exposed to oxidative stress (exposure to 70 mM $H_2O_2$). The highly positive correlation between oxidative stress tolerance and DJ-longevity when stored at 25°C, as well as a comprehensive overview of these characters has been published by Sumaya et al. (2017). A high variability and significant difference ($F$ = 36.62; $df$ = 39; $P \leq 0.0001$) in mean time survived by 50% of DJ populations ($MTS_{50}$) along the tested *H. bacteriophora* strains was recorded. The $MTS_{50}$ in DJ-populations under oxidative stress (70 mM $H_2O_2$) ranged from 3.2 $\pm$ 0.65 up to 22.46 $\pm$ 3.18 days. This measurement was highly correlated (R = 0.87, $P \leq 0.001$) with survival bioassays carried out under control conditions (0.0 mM $H_2O_2$, 25 °C). Natural isolates from Central Europe and Australia were found among the longest surviving materials. An overview of the determined $MTS_{50}$ under oxidative stress is presented by Sumaya et al. (2017).

Concerning virulence, all WT strains and inbred lines were able to infect *T molitor* larvae and significant differences were found among the $LD_{50}$ values ($K$ = 120.55; $df$ = 42; $P \leq 0.0001$). The $LD_{50}$ of the tested materials ranged from 1.4 to 30.5 DJs per insect. The most virulent strain against *T. molitor* larvae had a $LD_{50}$ of 1.4 $\pm$ 0.33. An overview of the $LD_{50}$ in all strains and inbred lines is depicted in Figure 2. New isolates with lower $LD_{50}$ values and better performance in other important traits (stress-resistance and DJ-longevity) are potential candidates for commercial product development.

**Selection of two contrasting materials for subsequent genetic analysis**

Genetic analysis is crucial for the generation of molecular markers for marker-assisted breeding. In this framework two strains with contrasting properties were chosen for subsequent genetic linkage and QTL analysis: i) DE2-IL1 (short DJ-survival, high virulence), and DE6-IL4 (long DJ-survival, low virulence). Both strains originated from wild type strains, inbred for more than eight self-fertilization cycles. The DJ-longevity phenotype of both inbred lines was confirmed by $MTS_{50}$ estimation under oxidative stress (70 mM $H_2O_2$) as shown in Figure 3.

**Crossing *H. bacteriophora* homozygous inbred lines for QTL analysis of recombinant inbred lines (RILs) and for generation of improved hybrids**

Genetic crosses were carried out with inbred lines to carry out QTL analysis. A description of the parental inbred lines and progeny is deposited in Table 1. All crosses were done following the report of Iraki and co-authors (2000). Following crossing, sets of highly homozygous recombinant inbred lines (RILs) were produced by self-fertilization starting with the hybrids. After genetic crossing, single progeny individuals were self-fertilized for more than 8 generations ($>F_8$) to produce recombinant inbred lines (hereafter, D2D6 RILs). These lines were chosen to be extensively genotyped by high throughput sequencing using the genotyping by sequencing (GBS) approach. All RILs were also characterized for DJ-longevity. Subsequently, genotype and phenotype were correlated by QTL (Fig. 5) and association analysis.

Table 1. *H. bacteriophora* inbred lines used for additional genetic crosses

| Hybrid name | Parent 1 | Phenotype | Parent 2 | Phenotype | Outcome |
|---|---|---|---|---|---|
| D2D6 | DE2-IL1 | low DJ-longevity High virulence | DE6-IL4 | high DJ-longevity low virulence | RILs |

**Variability in oxidative stress survival and desiccation tolerance along D2D6 RILs**

Phenotypic data for QTL and association analysis was generated by calculating the $MTS_{50}$ under 70 mM $H_2O_2$ of each RIL derived from the D2D6 cross. The DJs of the stress tolerant parent line (DE6-IL4) survived the longest time on the set ($MTS_{50}$ = 7.9 ± 1.3 days), whereas a lower $MTS_{50}$ value (5.1 ± 1.1 days) was determined for DE2-IL1, however, not the lowest among all lines. The RIL D2D6-42 survived the shortest time among all lines ($MTS_{50}$ = 3.7 days). Differences in $MTS_{50}$ among lines were significant ($F$ = 8.55; $df$ = 51; $P \leq 0.0001$). An overview of the RILs survival along the parental lines is shown in Figure 4.

**Production of hybrid strains combining high DJ-longevity and virulence by parallel genetic crosses**

Aside the crosses made for genetic studies (QTL analysis), a series of parallel crosses between strains or inbred lines having either high longevity or high virulence was carried out using candidate strains selected from the above described DJ-longevity and virulence survey. The 5 most virulent strains were crossed with the 15 strains with the highest DJ-longevity. A total of 75 hybrid progenies were produced and were left for at least 8 cycles of self-fertilization to create recombinant inbred populations. DNA from the inbreds was extracted for genotype analysis using molecular markers designed from SNPs associated with DJ-longevity and virulence. Hybrids and parents are summarized in Table 2.

Table 2. Parentals from high virulent strains (horizontally arranged) and strains with high DJ-longevity (vertically arranged), along with their cross hybrids denoted with the prefix HYB and the serial number of the cross.

| | | **Highly virulent strains** | | | | |
|---|---|---|---|---|---|---|
| | | PT4 | HU2 | PT1 | IL1 | IR3 |
| **Strains with high DJ-longevity** | HY1 | HYB1 | HYB16 | HYB31 | HYB46 | HYB61 |
| | IR2 | HYB2 | HYB17 | HYB32 | HYB47 | HYB62 |
| | HU2 | HYB3 | | HYB33 | HYB48 | HYB63 |
| | IT6 | HYB4 | HYB19 | HYB34 | HYB49 | HYB64 |
| | CN4 | HYB5 | HYB20 | HYB35 | HYB50 | HYB65 |
| | IL7 | HYB6 | HYB21 | HYB36 | HYB51 | HYB66 |
| | S-VI-MM8 | HYB7 | HYB22 | HYB37 | HYB52 | HYB67 |
| | IR1 | HYB8 | HYB23 | HYB38 | HYB53 | HYB68 |
| | TR2 | HYB9 | HYB24 | HYB39 | HYB54 | HYB69 |
| | S-VI-MM14 | HYB10 | HYB25 | HYB40 | HYB55 | HYB70 |
| | DE8 | HYB11 | HYB26 | HYB41 | HYB56 | HYB71 |
| | AU1 | HYB12 | HYB27 | HYB42 | HYB57 | HYB72 |
| | HY3 | HYB13 | HYB28 | HYB43 | HYB58 | HYB73 |
| | DE6 | HYB14 | HYB29 | HYB44 | HYB59 | HYB74 |
| | HU1 | HYB15 | HYB30 | HYB45 | HYB60 | HYB75 |

## Evaluating the genetic diversity of *H. bacteriophora* strains and RILs

### Genome-wide genotyping by sequencing (GBS)

Parallel to the phenotypic analysis, a subset of 28 D2D6 RILs, 11 wild type *H. bacteriophora* strains and 6 WT inbred ILs were analyzed by GBS as described by Elshire et al. (2011). The materials selected for the analysis were chosen according to their DJ-longevity (contrasting material Table 3). All *H. bacteriophora* lines and strains chosen for GBS were propagated in NGG media and harvested as described above. Clean DJs were used for DNA extraction with the peqGOLD Tissue DNA Mini Kit (PeqLab, Germany), according to the manufacturer's instructions. All sequencing steps were carried out by the company LGC Genomics GmbH (Berlin, Germany) according the GBS standard protocol.

Table 3. *Heterorhabditis bacteriophora* materials selected for genotyping by sequencing (GBS). For each material, its DJ-longevity under oxidative stress is depicted. Within each subset, contrasting materials are included. Materials with three different genetic backgrounds were analysed: WT strains, WT inbred lines, RILs and selected cross-progenies

| Type | Code | Longevity 25 °C/ 70 mM $H_2O_2$ |
|---|---|---|
| **WT Strains** | HY3 | High |
| | AU1 | High |
| | DE6 | High |
| | HU1 | High |
| | HU2 | High |
| | DE2 | low |
| | PT1 | low |
| | PT2 | low |
| | PT4 | low |
| | ENO1 | low |
| | PT3 | low |
| **WT Inbred lines** | AU1-IL1 | high |
| | HU2-IL1 | high |
| | DE6-IL4 | high |
| | PT1-IL1 | low |
| | IL3 | low |
| | DE2-IL1 | low |

(continued)

| Type | Code | Longevity 25 °C/ 70 mM $H_2O_2$ |
|---|---|---|
| RILs | D2D6-133 | high |
| | D2D6-24 | high |
| | D2D6-124 | high |
| | D2D6-125 | high |
| | D2D6-126 | high |
| | D2D6-135 | high |
| | D2D6-109 | high |
| | D2D6-99 | high |
| | D2D6-10 | high |
| | D2D6-84 | high |
| | D2D6-143 | high |
| | D2D6-144 | high |
| | D2D6-104 | high |
| | D2D6-114 | high |
| | D2D6-113 | low |
| | D2D6-111 | low |
| | D2D6-95 | low |
| | D2D6-128 | low |
| | D2D6-78 | low |
| | D2D6-71 | low |
| | D2D6-22 | low |
| | D2D6-21 | low |
| | D2D6-54 | low |
| | D2D6-93 | low |
| | D2D6-122 | low |
| | D2D6-94 | low |
| | D2D6-123A | low |
| | D2D6-123B | low |
| Selected progenies | HU2-IL1xPT1-IL1-Vir | (very) high |
| | HU2-IL1xPT1-IL1-LL+Vir | (very) high |
| | HU2-IL1xPT1-IL1 | high |

**_In silico_ analysis of GBS data and genotype-phenotype correlation analysis**

The objective of the GBS analysis is to find polymorphisms in large numbers among the tested materials. The targeted polymorphisms are single nucleotide substitutions in the genetic code (SNPs). After sequencing, GBS clusters harboring SNPs among the analyzed strains and lines were filtered according to the allele frequencies (reduction of minor SNP alleles) and variant call files (VCF) were generated. The obtained VCF were used to test the correlation between the lines SNP-genotype and existing longevity phenotype information using the software Tassel 5.0 (http://www.maizegenetics.net/tassel). SNPs with association to a specific longevity-related trait were filtered out after analyzing natural strains and RILs in separate runs. As a complementary approach, GBS clusters showing polymorphisms along the sequenced RILs were filtered and used for linkage analysis with the software package Joinmap 4.1 (https://www.kyazma.nl) with the Kosambi genetic mapping function with a minimum LOD score (logarithm [base 10] of odds) of 2.0. For QTL analysis, the linkage map generated by joinmap 4.1 was combined with RILs phenotypic data using the PlabQTL software (https://plant-breeding.uni-hohenheim.de/software.html). PlabQTL was run including test for additive and dominance effects (no test for residuals). GBS clusters flanking QTL were selected for further analyses.

**Determination of SNPs with correlation with longevity and virulence**

Illumina paired end-sequencing yielded a total of 80.000 GBS clusters (64 bp sequence stretches). After filtering out minor SNP alleles, 1.126 clusters resulted polymorphic either in natural strains or in RILs. Further on, BLAST analyses against the public repositories yielded 380 clusters with high homology to the latest version of the *H. bacteriophora* genome draft. Additionally, 230 clusters presented high homology to the NCBI (nr) database. For further analyses, variant call files (VCF) were generated for two categories of sequenced individuals: i) natural strains and inbred lines, ii) RILs and parental inbred lines.

The phenotype and genotype information from the previously analyzed *H. bacteriophora* strains and inbred lines was combined by association analysis. SNP variant calls from wild type lines and strains were analyzed under an allele frequency threshold of 0.05 for the minor SNP allele. With this approach, a total of 1075 SNPs were analyzed using the Tassel 5.0 software. Phenotypic data from the DJ-survival ($MTS_{50}$ and $MTS_{10}$) of 17 strains and inbred lines were combined with the respective SNP alleles. Phenotype information has been published by Sumaya et al. (2017). Survival time from four treatments was considered: i) 25 °C - control, 25 °C - oxidative stress, 7 °C - control, and 7 °C - oxidative stress. After data analysis, association to at least one of the traits was shown by five markers. Marker details and associations are depicted in Table 4.

Table 4. GBS-clusters showing SNPs with association to DJ-longevity related traits in *Heterorhabditis bacteriophora* natural strains and inbred lines. For each SNP, associated traits are depicted along their P-value. Homology hits with previously sequenced RNA-seq transcripts and contigs of the *H. bacteriophora* current genome draft are shown.

| | Trait | | | BLAST homology hits | |
|---|---|---|---|---|---|
| **Marker** | **Parameter** | **Conditions** | **p-value** | **RNA-seq transcripts** | ***H. bacteriophora* genome draft** |
| SC00002607 | $MTS_{50}$ | 25 °C - 70 mM $H_2O_2$ | 2.78E-05 | EN-Hb_oxid-51278 | contig1336 |
| | $MTS_{10}$ | 25 °C - 70 mM $H_2O_2$ | 7.62E-04 | EN-Hb_oxid-51278 | contig1336 |
| SC00003550 | $MTS_{50}$ | 25 °C - 70 mM $H_2O_2$ | 6.69E-06 | | |
| | $MTS_{10}$ | 25 °C - 70 mM $H_2O_2$ | 1.32E-04 | | |
| SC00004013 | $MTS_{50}$ | 7 °C - 70 mM $H_2O_2$ | 4.24E-04 | | |
| | $MTS_{10}$ | 7 °C - 70 mM $H_2O_2$ | 8.31E-04 | | |
| SC00004647 | $MTS_{50}$ | 25 °C - 70 mM $H_2O_2$ | 2.78E-05 | | contig1265 |
| | $MTS_{10}$ | 25 °C - 70 mM $H_2O_2$ | 7.62E-04 | | contig1265 |
| SC00006203 | $MTS_{50}$ | 25 °C - 70 mM $H_2O_2$ | 2.78E-05 | | contig1190 |
| | $MTS_{10}$ | 25 °C - 70 mM $H_2O_2$ | 7.62E-04 | | contig1190 |

**QTL analysis determines SNP with high association with DJ-longevity**

A QTL analysis was carried out using SNPs recorded for the 28 D2D6-RILs analyzed by GBS aside the parent lines DE2-IL1 and DE6-IL4. Genotype data were combined with RILs phenotype data described in the previous sections (see Fig. 4). Additionally, desiccation tolerance data from the same RILs were included (data not shown). For linkage analysis, 321 SNPs for the parental and the RILs were chosen. After filtering for all SNP minor alleles, 63 SNPs were used for the construction of a linkage map using the Joinmap software. A low resolution linkage map with five major linkage groups was obtained including 53 SNPs. The developed genetic map covered 553 centimorgan (cM). Thereafter, phenotype and genotype were correlated by QTL analysis (Fig. 5). The phenotypic data from three different oxidative trials and one desiccation trial (each consisting of three technical replicates) was used independently for the QTL calculation. Seven QTL with profile LOD score above 2.0 were determined in three linkage groups. Basic QTL parameters are deposited in Table 5. SNPs in the vicinity of the QTL were later chosen for the design of PCR-base genotyping markers.

Table 5. Oxidative stress- ($MTS_{50}$) and desiccation-tolerance QTL detected in *Heterorhabditis bacteriophora* linkage groups determined using polymorphic SNPs. For each QTL, the left and right markers are depicted along with the total LOD score of the QTL. Positive additive effects indicate DE6-IL4 as strong parent. Negative additive effects indicate DE6-IL4 as weak parent, for the given loci

| Trait-trial | LG | Position | Left SNP Marker | Right SNP Marker | LOD | Additive effect |
|---|---|---|---|---|---|---|
| $MTS_{50}$-$H_2O_2$-2 | 1 | 187 | SC00005330 | SC00004141 | 2,51 | 3,75 |
| $MTS_{50}$ $H_2O_2$-2 | 1 | 213 | SC00004634 | En_Oxid-8385 | 2,04 | -2,01 |
| $MTS_{50}$ $H_2O_2$-2 | 1 | 314 | SC01010026 | SC00014329 | 3,19 | -4,47 |
| $MTS_{50}$ $H_2O_2$-1 | 2 | 6 | SC01100604 | SC00061659 | 5,36 | 0,70 |
| $MTS_{50}$ $H_2O_2$-1 | 3 | 59 | SC00022876 | SC00024776 | 7,52 | -0,69 |
| $MTS_{50}$ $H_2O_2$-Avg | 2 | 0 | SC00038306 | SC00100604 | 1,76 | 0,56 |
| Desiccation -1 | 1 | 207 | SC00006203 | SC00004634 | 5,02 | 8,60 |

**Transfer of information from genome-wide SNP analysis into PCR-based markers**

The GBS analysis was done with a limited number of *H. bacteriophora* strains and RILs. The next step of the research was to transfer this information to design PCR-based SNP markers and to test a wider number of WT strains (40), D2D6-RILs (50), and all hybrids (75) generated from candidate strains (Table 2). Once all strains and RILs were genotyped, the correlation between genotype, DJ-longevity and virulence was tested to select KASP markers with the most predictive power. These markers were re-tested in the cross hybrids.

Sequence information from GBS was converted into PCR markers for genotyping assays. A set of 30 candidate SNPs derived from the QTL and association analyses described above was chosen for the SNP detection assays by PCR (Table 6). The technique known as KASP (Competitive Allele Specific PCR) was chosen for this purpose. For KASP marker primer design, all pre-selected sequences were send to the commercial partner LGC genomics (UK). KASP-PCR amplifications were done starting from 2-3μl DNA (10-15ng) of each genotyped nematode material using the markers (primers) by following the PCR-profile: 94 °C-15min; 10 cycles of 94 °C-20 sec, 61 °C-1 min (0.6 °C drop each cycle) and 30 cycles of 94 °C-20 sec, 55 °C-1 min. Resulting signals were analyzed with the StepOne software (genotyping mode) of Applied Biosystems for allele discrimination. For each genotyping round, KASP assays with no DNA were used as negative controls.

**KASP genotyping in WT strains confirms SNPs with association to DJ-longevity**

Out of a total of 30 KASP markers (Table 6), 23 markers where polymorphic in the 40 wild type strains. To validate the KASP results, the GBS genotypic data of the 28 D2D6-RILs and their parents was compared in 5 randomly selected KASP markers. A total of 140 data points were compared between GBS and KASP assays. From GBS clusters registered as homozygous, 94 out of 94 KASP readings were consistent with the expected genotype. In 11 cases, where the GBS genotype for the given RILs was regarded as heterozygous, the KASP genotype was registered as homozygous for one of the parental alleles. In summary, 90% concordance was observed for all amplified SNPs.

The genotypes derived from all the KASP markers were analyzed for significant phenotypic differences in survival ($MTS_{50}$) along 50 D2D6-RILs and 40 WT strains and inbred lines via ANOVA. For the D2D6 RILs, the genotypic data were combined with the $MTS_{50}$ of the RILs under oxidative stress from two experimental trials and desiccation the survival. For wild type strains and inbred lines, the genotypic data were combined with the $MTS_{50}$ under two temperatures (25 °C and 7 °C), and two conditions (0.0 and 70 mM $H_2O_2$) published by Sumaya et al (2017). Concerning virulence, the $LD_{50}$ (number of DJs needed to kill 50% of a host population) of the WT-strains against mealworm (*Tenebrio molitor*) was used. Among the 40 wild type strains and inbred lines, twelve markers showed correlation between the genotype and at least one the $MTS_{50}$ parameters measured. Two of these markers (SC00004554 and SC00010093) showed also high correlation with the $LD_{50}$ against *T. molitor*. A detailed overview of the significant markers for the 40 wild type strains and inbred lines is given in Table 7.

Table 6. Overview of designed KASP markers for the genotyping of *Heterorhabditis bacteriophora* strains and inbred lines. Allele variants with the specific fluorophore are provided along with sequence source of the SNP.

| Marker Name | Allele FAM | Allele HEX |
|---|---|---|
| SC00002607 | G | T |

(continued)

| Marker Name | Allele FAM | Allele HEX |
|---|---|---|
| SC00003427 | G | T |
| SC00003742 | G | A |
| SC00004554 | G | A |
| SC00004647 | C | T |
| SC00004911 | T | C |
| SC00005459 | C | T |
| SC00005496 | A | T |
| SC00005718 | A | T |
| SC00006203 | C | T |
| SC00006291 | T | G |
| SC00010093 | A | G |
| SC00010669 | C | G |
| SC00011215 | T | C |
| SC00011443 | C | T |
| SC00012176 | G | A |
| SC00012917 | G | C |
| SC00013602 | G | A |
| EN-Hb_oxid-05173 | G | T |
| EN-Hb_oxid-08385 | C | G |
| EN-Hb_oxid-11688 | G | C |
| EN-Hb_oxid-26008 | G | A |
| EN-Hb_oxid-41943 | C | T |
| EN-Hb_oxid-45902 | G | C |
| EN-Hb_oxid-48911 | G | A |
| EN-Hb_oxid-51540 | T | C |
| EN-Hb_oxid-56842 | A | G |
| EN-Hb_oxid-56985 | G | A |
| EN-Hb_oxid-58060 | C | T |
| EN-Hb oxid-58700 | C | T |

Table 7. Differences in the DJ-longevity and virulence between homozygous genotypes of the reference and alternative SNPs of 12 KASP markers along 40 *Heterorhabditis bacteriophora* wild type strains and inbred lines. The differences between mean $MTS_{50}$ of the homozygous genotypes (days) are depicted as absolute values. Differences in virulence ($LD_{50}$) are depicted as number of DJs. For DJ-longevity, phenotypic data from two treatments (0 and 70 mM $H_2O_2$) under two temperatures (25 °C and 7 °C) were analyzed. For virulence $LD_{50}$ against *Tenebrio molitor* was used as phenotypic trait. Significance of the differences between genotypes was assessed via ANOVA and Tukey's HSD test ($P \leq 0.05$) and is indicated with stars.

| Marker name | $LD_{50}$ difference *T. molitor* | $MTS_{50}$ 25°C Difference (days) | $MTS_{50}$ 25°C 70 mM $H_2O_2$ Difference (days) | $MTS_{50}$ 7°C Difference (days) | $MTS_{50}$ 7°C 70 mM $H_2O_2$ Difference (days) |
|---|---|---|---|---|---|
| EN-Hb_oxid-05173 | | 0.8 | 3.7* | 5.1 | 1.6 |
| EN-Hb_oxid-56985 | | 0.3 | 5.2* | 8.7 | 2.2 |
| SC00003427 | | 9.2* | 3.4 | 6.2 | 2.1 |
| SC00004554 | 1.26* | 6.6* | 2.4 | 1.3 | 1.6 |

(continued)

| Marker name | $LD_{50}$ difference *T. molitor* | $MTS_{50}$ 25°C Difference (days) | $MTS_{50}$ 25°C 70 mM $H_2O_2$ Difference (days) | $MTS_{50}$ 7°C Difference (days) | $MTS_{50}$ 7°C 70 mM $H_2O_2$ Difference (days) |
|---|---|---|---|---|---|
| SC00004647 | | 7.8* | 7.3* | 10.7* | 4.5* |
| SC00004911 | | 2.6 | 4.4* | 13.6* | 3.7 |
| SC00005330 | | 5.2 | 8.2* | 6.2 | 2.6 |
| SC00006203 | | 7.3* | 5.9* | 13.2* | 4.6* |
| SC00010093 | 1.31* | 6.0* | 0.7 | 3.3 | 0.3 |
| SC00011215 | | 11.0* | 10.2* | 8.9 | 5.2* |
| SC00012917 | | 7.5* | 0.3 | 1.0 | 0.6 |
| SC00013602 | | 0.7 | 4.5* | 9.7* | 3.1* |

**Use of the SNP-Marker for selection of hybrids with better DJ-longevity and virulence**

KASP genotypes were cross checked along the hybrids depicted in Table 2 with the objective to choose a material for subsequent characterization. The HYB56 hybrid was selected for further tests, including performance in the field against the WCR, persistence and longevity. Concerning significant KASP markers 8 out of 12 markers (66%) resulted distinctive between HYB56 and the commercial EN01 strain. The polymorphic markers SC00006203, SC00005330, SC00004141 and SC00004634 were found in the vicinity of DJ-longevity QTL (see Tables 4 and 5). Moreover, markers that combined correlation with virulence and DJ-longevity (SC00004554 and SC00010093) were as well polymorphic between HYB56 and EN01. The KASP genotypes of the subset of markers had the highest association with DJ-longevity and virulence including their genotype, along with four additional polymorphic markers extracted from relevant transcripts and QTL vicinity (Table 8). Flanking sequences of SNPs for KASP probe design are deposited in Table 9.

Table 8. Genotypes of KASP markers associated with DJ-longevity along the commercial *H. bacteriophora* strain (EN01) and the longer living hybrid HYB56.

| Marker Name | Longevity Sign. | Virulence Sign. | EN01 | HYB56 |
|---|---|---|---|---|
| SC00003427 | * | | T/T | G/G |
| SC00004141 | | | A/A | T/T |
| SC00004554 | * | * | G/G | A/A |
| SC00004634 | | | C/C | T/T |
| SC00004647 | * | | C/C | T/T |
| SC00004911 | * | | C/C | C/T |
| SC00005330 | * | | G/G | A/A |
| SC00006203 | * | | C/C | T/T |
| SC00010093 | * | * | G/G | A/A |
| SC00011215 | * | | T/C | T/C |
| SC00012917 | * | | C/C | G/G |
| SC00013602 | * | | A/A | A/A |
| EN-Hb_oxid-05173 | * | | G/G | G/G |
| EN-Hb_oxid-11688 | | | G/G | C/C |
| EN-Hb_oxid-26008 | | | A/A | G/G |
| EN-Hb_oxid-56985 | * | | G/G | G/G |

Table 9. Sequences used for KASP assays design for the identification of *H. bacteriophora* SNPs by PCR.

| SEQ ID | Marker | Sequence for probe |
|---|---|---|
| 1 | SC00003427 | TTATCAAGTAAATAAAGTTCGTCTATTTTTATTAAGATTTTCTCACTAAAGTGATAAGTATGTTG**[T/G]**AGTTCTTGATTAGTATTAATTAACAGCGATTAAATGCCAGAGAGGCAATAAACGCTGTGTAAACCCACATTAATTTAGCTTTTTCTATTCACAGATTC |
| 2 | SC00004141 | TACATACTTGCATTAAATGGAACAAAGTGCTCATCAATGTGCATTTAGTATTTACATCTATGTGTATGAAATGTG**[A/T]**CATCTGTATATTGTGCGAACTTAACAAAGAAAGACTTATTGAGGTCATTTTTATATACATGGTGTCCACGATAAAAGGACCTATTTGACAAGTTTTATAACT |
| 3 | SC00004554 | AATTAAACCGCAGATGACCGAGCCAGGGGTGAGTTTTTCGGTGCACTTCGATGTGAGTTTGAAGACTGCGAG**[G/A]**GATGTAAGTTTACTGGTGAGTTTTCCTTTATATTTTTTTTCAGTACTCTCCTGAGCCGAGGCGTTTGCCTCAGTGCTCTTTTCCCACCTCC |
| 4 | SC00004634 | ATCTTTTAGGAAGTACAAAAGATGTATAATTTATTTACTAGTAATAATTCGCCACGTTCTTCTACTATCCATGTTGATGTTGTCA**[C/T]**TATGTTTAAGCACTTGATAGGTATATGGATACACATCTGAGATTTCGTGTCATTTACTTTACCCCGGTTACTTTTCGGGCTATTTTATACCCTT |
| 5 | SC00004647 | AACTTAGTAATAAAATTCGTAAAAATTATTTTATGCTTACATTCACTCCTATGGACTTCTACATAGAAGGCTTC**[C/T]**GATGAGCGGGGAATAAGCCCTCGCTGTCCAGTGGCAATATTCATCGCATCCAGTGAACAATCCCTTTAATATGTGAAACTTAAAGTTGG |
| 6 | SC00005330 | CTATTACTACTACTATTACTATTATCAAGTTGAGTTAAATTAATAAAGGTGAAAATATTGTGGCATTATTTTTGACATGCCTGTGGTTTGAATCACT**[G/A]**CTTTTTTTTATCATGATTTTTATTCTAGAATGGTACCAAATTGTATAGTAAAGGCGAAGAACGAAAGGAAGCGAGTATACGTCGGTGAAGAATTATGTGAATGTGCTGATCGAAGCAGCCTATTCTTCGTATTA |
| 7 | SC00006203 | TCACCGAAATATTGTGGTAGAAGTTAGCGTGAGAAGTTGGACTCATATTAGTG**[C/T]**TATTCATCGAATGGACATGGGAAAACAGTTACTCAGATAACTGTTCCTTTGCCCTGTGAATAAGGGCAGATTTAATCTTACGGTTACTGGTCTTCATGGCTGAACAACTT |
| 8 | SC00010093 | GTGGCGAGAAGAAAGAATAAGTATTATTTGAAAGATCAATATCCATTAATATGAGTGAACAATTGAATAGGACAATAGTTAAATGATAGAAGGTTTAACTCAATGGTTAA**[G/A]**TTTAAAAAGATAAGGGAACTACTTCAGACAGGTCTTCGGCGCACGGAATCGGC |
| 9 | SC00012917 | TTGTTCCATTGTTCAAAAACATTGTAATACTGTCAACTATTGCTTGGAACATGTTCTAGAATAATGGTTCATTGGC**[C/G]**ATTTGCCGTCATTAGTAATGTTAAAATAGTTTTAATCTGTAGTGGATTTGTGGCGGACGCAGTGGTTGATGCATCAGAATTGTTCCA |
| 10 | EN-Hb oxid-11688 | GCTTCATGGTTTTAGCCATACAATCGATGATGCCATTATAGGCATTCATGGTTTGTAGTCTAGCCTTGACTGTGTCCAGCGGATGTCCAACGAGAAGGCCTGCTCCTCCTAACATATTAAAGGTCATAGCCGATCTCGCCCGCCCCATTTTCTCAGCGTAAATAATCAATCGAACTACGAGAGGTCAACCAAACGGCTG**[G/C]**ACTGCTTATTGACAGTTTTGCTGTTAGCGTTCTCGTATTTTATATTTGCACCTCATTATATTTTAGTTTGTCTAATTAAATATATGAACTAATTGATAAATAAATAGGTTCTTACTTC |
| 11 | EN-Hb_ oxid-26008 | CAAAAATTCCGATCAACATACTTTATACATTTTATCGTTATGAAGTCATTTATTCATTGACTGAGAAAAATATAAGTGAAGAGCCACTAATTAATCGATATATAAGTAGCTACAAGATTGATTTTTAATACTATTGTAAATAATAATTAGTTAAATGCATTGTAGCAAATTAAAA**[A/G]**CTAATGATATCTAAGAAAATCCCGGAAGAAAAGGATACGAAACGGTCATCTAACAACGCTATAATAATTATGCAGTTTTAATTTTCTTGCTATTAAAAAATCGTAACAATAACATTGATACATATATATCGATTAATTAGTGGCTCTTCACTTATATTTTTCTCAGTCAATGAATAAATGACTTCATAACGATAAAATGT |

**Virulence and persistence of HYB56 and HYB56-IL73**

Based on phenotypic and genotypic information, the hybrid HYB56 and the daughter inbred line HYB56-IL73 were chosen for evaluation of longevity and virulence-related parameters in the laboratory.

Bioassays against mealworms (*Tenebrio molitor*) are a standard test for virulence testing after large-scale production at e-nema GmbH. The commercial strain EN01 along with the hybrid and inbred line HYB56 and HYB56-IL73 were evaluated after production in liquid culture. Sand-filled Petri dishes containing 40 insect larvae were inoculated with 80, 200, 400, 800 and 2000 DJs per dish and incubated at 25 °C for 7 days (as described in previous sections). Four replicates were done with each nematode amount for EN01, HYB56 and HYB56-IL73 in parallel. Insect mortality data were used to calculate the $LD_{50}$. Parallel to *T. molitor* assays, biotests containing 20 *Diabrotica v. virgifera* larvae were set up in sand (10% moisture) with the addition of a single maize seedling (cultivar Ronaldinho). Petri dishes were inoculated with 100, 200, 400, 800, 1600, and 3200 DJs per assay. The mortality of the Western Corn Rootworm was used to calculate the $LD_{50}$.

On *T. molitor,* significant differences were observed among the $LD_{50}$ ($F = 5.09$; $df = 4, 44$; $P \leq 0.002$) as depicted in Figure 6. Against *T. molitor,* the lowest $LD_{50}$ value was observed for HYB56-IL73 (5.1 ± 0.37 DJs per insect). This value was significantly lower than the $LD_{50}$ of the commercial strain EN01 (14.4 ± 2.7 DJs per insect). An intermediate $LD_{50}$ value was recorded for HYB56 (10.54 ± 1.17 DJs per insect). Concerning virulence against *D. v. virgifera* the

lowest $LD_{50}$ was observed for HYB56-IL73 (8.2 ± 1.7 DJs per insect) whereas the highest value was determined for EN01 (24.8 ± 7.1 DJs per insect).

For assessment of persistence, EN01, HYB56 and HYB56-IL73 DJs were tested in potted soil assays using DJ application quantities deduced from the commercial field dose of $2 \times 10^9$ DJs $ha^{-1}$. Two liter pots (15 × 10 × 13cm) were supplied with a layer of stones (150 g) in the bottom and filled with soil (45% moisture). Three maize seeds (hybrid Ronaldinio, KWS, Germany) were inserted to each pot and seven days later were thinned out to continue with one plant per pot. Nematodes were inoculated on the soil surface suspended in 10 ml of Ringer's solution. The application dose per pot was calculated from the number of DJs applied ($2 \times 10^9$ DJs $ha^{-1}$) in a field sown with 75.000 plants per hectare. Accordingly, pots were inoculated with ~26.600 DJs to simulate the field conditions. Five replicates were done per nematode line and the pots were randomly distributed. The experiment was carried at 24 ± 2°C, 16:8 (light:dark) photoperiod and 65 ± 10 RH. Persistence was assessed by baiting nematodes with either *D. v. virgifera* or *T. molitor* over a period of 21 days after inoculation using adapted Falcon tubes with 200 μm sieve to impair the insect larvae to leave and the DJs to penetrate. Tubes were dug into the soil and left for seven days and then insect mortality was recorded. Dead insects were individually checked for luminescence of the symbiotic bacteria under a luminometer in order to confirm the infection by nematodes.

The most drastic differences in percentage of infected insects between strains and doses were observed after 21 days of DJ application (Figure 7). Against *D. v. virgifera*, the highest percentage infection was observed for HYB56-IL73 (32.0%), whereas the lowest percentage infection was observed for the commercial strain EN01 (8.1%). Against *T. molitor,* the highest infection percentage was determined for HYB56-IL73 (37.0%), whereas the lowest infection percentage was determined for EN01 (10%). Thus, at least a 3-fold reduction on the DJ dose (compared to EN01) to kill 50% of a population of WCR larvae after application has been achieved by combining the genotype and phenotype selection in *H. bacteriophora.* Virulence over time (persistence) of HYB56 and HYB56-IL73 is superior to the commercial strain EN01.

**Survival and virulence of post-formulated DJs**

DJs from HYB56 and HYB56-IL73 together with the strain EN01 were produced in liquid culture, washed, and formulated in powder at a density of $1 \times 10^6$ DJ $g^{-1}$. The formulated nematodes were packed in zip locked perforated plastic bags (5g per bag) and were stored at 6°C for 30, 45 and 63 days. Formulated DJs were then re-suspended in water (1g powder per 200 ml water), nematodes were washed-off from the powder and stored in Ringer solution at 15°C in cell culture bottles for maximum 1 week. Longevity was tested by testing oxidative stress tolerance at $H_2O_2$ concentrations of 0, 40, 80, and 100 mM $H_2O_2$. Three replicates per DJ line and $H_2O_2$ dose were set up in each assay and repeated three times. DJ survival was recorded 12 days after stress induction by counting living and dead individuals in a counting chamber (20 μl aliquots from each assay). The $H_2O_2$ concentration for 50% DJ mortality was subsequently determined.

After 30 days of cold storage in formulation, the highest $H_2O_2$ tolerance ($H_2O_2$ concentration for 50% DJ mortality) was determined for HYB56 (79±4 mM) whereas 50% of the EN01 DJs died at a concentration of 43±3 mM $H_2O_2$ (Figure 8). This large difference between both strains was conserved also after 45 days of storage. At this time point, 50% of the HYB56 and EN01 DJs died under 78+3 mM and 47+3 mM $H_2O_2$, respectively. The differences in $H_2O_2$ -tolerance between both strains were significant (F = 217.7, *df*=1, $P \leq 0.05$).

For virulence-testing of post formulated DJs, the standard virulence test against mealworm was performed as described. Seven days after assay start, the mortality of the mealworms was assessed and the $LD_{50}$ was calculated. After 30 days of storage, $LD_{50}$ values of 14.1 ± 1 and 7.9 ± 0.8 DJs per insect were determined for EN01 and HYB56, respectively. After 45 days, the $LD_{50}$ value increased for both strains (the virulence decreased). At this time point, the EN01 $LD_{50}$ value increased to ~32 ± 6.3 DJs per insect whereas HYB56 kept a lower $LD_{50}$ (11.3 ± 1.7 DJs per insect) (Figure 9). The differences between HYB56 and EN01 resulted statistically significant for all time points. ($F$ = 13.92; $df$ = 1, $P \leq 0.05$; 30 days), ($F$ = 19.61; $df$ = 1, $P \leq 0.011$; 45 days) and ($F$ = 100.3; $df$ = 1, $P \leq 0.008$; 63 days). The hybrid strain is at least 1-fold more tolerant to $H_2O_2$ than the commercial strain and is at least 1.8-fold more effective against *T. molitor* after formulation and long-time storage.

**Performance of HYB56 under field conditions with a natural WCR population**

The hybrid HYB56 was evaluated in field trials parallel to the commercial strain EN01. DJs of both strains were produced in large volume fermenters (500 and 3.000 Liters) and were harvested and formulated according to the standard e-nema GmbH procedure. Natural WCR populations are unevenly distributed in the field.

Field trials were carried out in Austrian locations (Styria). Nematodes were applied in maize fields naturally infested with WCR at Unterschwarza (hereafter field A) and Lichendorf (hereafter field B) at $2 \times 10^9$ DJs $ha^{-1}$ in April into the furrow with 200 liters of water per hectare with a sowing machine (Monosem 4) equipped with injectors for nematode application. WCR control was compared with an untreated control and the chemical insecticide Belem (Cypermethrin 0,8 % granules, at 12 kg $ha^{-1}$). Plots consisted of 30 rows of 30 m length for all treatments. The number of emerged beetles was determined by using 8 photoeclectors per treatment each covering 5 plants at 100 days after application (end of July). The plants damage level was registered at plant maturity according to the degree of stem inclination. Catagories assessed were a. plants lodged that are not possible to harvest, b. plants with more than 30° inclined stems, c. plants with less than 30° inclined stems (gooseneck syndrome) and healthy plants.

The number of emerging beetles per plant supports the better performance of HYB56 (Figure 10). In both fields A and B, the HYB56 substantially reduced the number of emerging insects compared to the untreated control and the chemical treatment and outperformed strain EN01 as well. Plant damage level are summarized in Figure 11. In both fields, damage was lower in the nematode treated area compared to control and chemical treatment. In field A no difference was recorded between the two nematode strains whereas in field B the genetically improved HYB56 outperformed strain EN01. The proportion of plants with gooseneck syndrome in plots treated with HYB56 (1.1%) was 4-fold lower than the proportion observed in plots treated with EN01 (4.8%).

Persistence of nematodes in the fields was also assessed by soil baiting using the Falcon tube method as previously described. Baiting was done 9 weeks after nematode application. Each Falcon tube received 18 g of soil, ten *T. molitor* larvae and one maize seedling. Tubes were buried at 10 cm deep in the soil and left in soil contact for 10 days. Baiting was done at five replicates per treatment. At the end of the baiting period, tubes were collected and the infection of *T. molitor* larvae was recorded.

The results confirm the higher persistence of the HYB56 hybrid. The nematodes were applied at 1 and $2 \times 10^9$ DJs $ha^{-1}$. The percentage of infection after baiting with *T. molitor* are presented in Figure 12. EN01 DJs controlled 28% and 33% of the insects at 1 and $2 \times 10^9$ DJs $ha^{-1}$, respectively, whereas the HYB56 strain was 2-fold more effective than EN01 nine weeks after DJ application (64% infected insects).

**Performance of HYB56 under field conditions with plants infested with WCR eggs**

In order to reduce the variability in the distribution of WCR populations, trials were performed on non-infested fields and plants were inoculated with WCR eggs. This approach can help to reduce the variability of the results. Field trials were performed in Hungary by CABI (www.cabi.org).

The field had been ploughed in autumn of the previous cropping season and was tilled and harrowed in April. Sowing was carried out in April together with application of DJs of strains EN01 and HYB56 at $2 \times 10^9$ DJs $ha^{-1}$ as performed during trials in Austria (untreated control and Cypermethrine 0,8 % granules at 12 kg $ha^{-1}$). Four plots of 6 rows (4.5 x 20 m each) were used per treatment. *D. v. virgifera* eggs (500 per plant) were infested in treated and untreated plots (control) among the 4 middle rows on 2 x 6 successive maize plants ($\approx$ 1.2 meters), randomly chosen. Eggs were applied into two 100 to 140 mm-deep holes at a distance of 110 to 190 mm from both sides of the plant when the plants were at 1 to 2 leaf stage.

Reduction of the WCR population (Abbott corrected reduction against the untreated control) was assessed by covering 5 plants with cages and emerging adults were collected starting in the beginning of May until no more adults emerged (end of August) . HYB56 reduced the WCR population by 65,8% (Figure 13). Efficacy of 28.0% was determined for plots treated with EN01, whereas the efficacy of the chemical treatment with Cypermethrin (Belem) reached only 42% control. Root damage (reduction of root damage caused by WCR larvae compared to untreated control) was assessed with up-rooted plants using the IOWA scale (Hills and Peters, 1971). Again, the HYB56 strain outperformed strain EN01 and the chemical treatment (Figure 14).

All laboratory and field evaluations applied with the *H. bacteriophora* hybrid HYB56 and the inbred line HYB56-IL73 support the conclusion that the combination of the phenotypic characterization and genotyping information lead to the selection of a nematode strain with robust improvement in DJ-longevity, virulence and persistence in comparison to the commercial strain EN01. The markers detecting SNP alleles associated to DJ-longevity and virulence present in the hybrid HYB56 and HYB56-IL73 are the result of extensive genetic research. These molecular markers are a crucial tool to detect nematode cross progenies with better performance for use in biocontrol control.

**LITERATURE**

Bai X, Adams BJ, Ciche TA, Clifton S, Gaugler R, Hogenhout SA, Spieth J, Sternberg PW, Wilson RK, Grewal PS (2009) Transcriptomic analysis of the entomopathogenic nematode Heterorhabditis bacteriophora TTO1. BMC Genomics 10: 205.

Bai X, Adams BJ, Ciche TA, Clifton S, Gaugler R, Kim KS, Spieth J, Sternberg PW, Wilson RK, Grewal PS., et al.

(2013). A lover and a fighter: the genome sequence of an entomopathogenic nematode Heterorhabditis bacteriophora. PLoS One 8: e69618.

Ehlers R-U (2001) Mass production of entomopathogenic nematodes for plant protection. Applied Microbiology and Biotechnology 56:623-633.

Ehlers R-U, Oestergaard J, Hollmer S, Wingen M, Strauch O. (2005). Genetic selection for heat tolerance and low temperature activity of the entomopathogenic nematode bacterium-complex Heterorhabditis bacteriophora-Photorhabdus luminescens. Biocontrol 50, 699-716.

Elshire RJ, Glaubitz JC, Sun Q, Poland JA, Kawamoto K, Buckler ES, Mitchell SE (2011) A robust, simple genotyping-by-sequencing (GBS) approach for high diversity species. PloS One 6: e19379.

Grewal PS, Wang X, Taylor RAJ (2002). Dauer juvenile longevity and stress tolerance in natural populations of entomopathogenic nematodes: is there a relationship? International Journal for Parasitology 32: 717-725.

Grewal PS, Ehlers R-U, Shapiro-Ilan DI (Eds) (2005). Nematodes as Biocontrol Agents. Wallingford, UK, CAB International Publisher.

Grewal PS, Peters A (2005). Formulation and quality control of entomopathogenic nematodes. In: Grewal, P.S., Ehlers, R.-U. & Shapiro-Ilan, D. (Eds). Nematodes as Biocontrol Agents. Wallingford, UK, CAB International Publisher, pp. 79-90.

Guo W, Yan X, Zhao G, Han R (2013) Efficacy of entomopathogenic Steinernema and Heterorhabditis nematodes against white grubs (Coleoptera: Scarabaeidae) in peanut fields. Journal of Economic Entomology 106: 1112-1117.

Hills TM, Peters DC (1971) A method of evaluating post planting insecticide treatments for control of Western Corn Rootworm larvae. Journal of Economic Entomology 64: 764-765.

Iraki N, Salah N, Sansour MA, Segal D, Glazer I, Johnigk SA, Hussein MA, Ehlers R-U (2000) Isolation and characterization of two entomopathogenic nematode strains, Heterorhabditis indica (Nematoda, Rhabditida), from the West Bank, Palestinian Territories. Journal of Applied Entomology 124:375-380.

Johnigk, S-A, Ehlers, R-U (1999). Endotokia matricida in hermaphrodites of Heterorhabditis spp. and the effect of the food supply. Nematology 1: 717-726.

Kiss J, Komaromi J, Bayar K, Edwards CR, Hatala-Hsellér I (2005) Western Corn Rootworm (Diabrotica virgifera virgifera LeConte) and the crop rotation system in Europe. In: Vidal S, Kuhlmann U, Edwards CR (Eds) Western Corn Rootworm: Ecology and Management. Wallingford, UK. CAB International Publisher, pp. 189-220.

Krysan JL, Smith RF (1987) Systematics of the virgifera species group of Diabrotica (Coleoptera: Chrysomelidae: Galerucinae). Entomography 5: 375-484.

Long SJ, Richardson PN, Fenlon JS (2000) Influence of temperature on the infectivity of entomopathogenic nematodes (Steinernema and Heterorhabditis spp.) to larvae and pupae of the vine weevil Otiorhynchus sulcatus (Coleoptera: Curculionidae). Nematology 2:309-17.

Mukuka J, Strauch O, Ehlers R-U (2010a) Variability in desiccation tolerance among different strains of the entomopathogenic nematode Heterorhabditis bacteriophora. Nematology 12, 711-720.

Mukuka J, Strauch O, Nellas Sumaya NH, Ehlers R-U (2010b) Improvement of heat and desiccation tolerance in Heterorhabditis bacteriophora through cross-breeding of tolerant strains and successive genetic selection. BioControl 55:511-521.

Poinar GO (1975) Description and biology of a new insect parasitic rhabditoid, Heterorhabditis bacteriophora n. Gen., n. Sp. (Rhabditida: Heterorhabditidae n. Fam.). Nematologica 21:463-470.

Regeai S, Dolan K, Fitzpatrick DA, Browne J, Jones J, Burnell A (2009) Novel primers for the amplification of nuclear DNA introns in the entomopathogenic nematode Heterorhabditis bacteriophora and their cross-amplification in seven other Heterorhabditis species. Molecular Ecology Resources 9: 421-424.

Shapiro-Ilan DI, Hazir S, Lete L (2015) Viability and virulence of entomopathogenic nematodes exposed to ultraviolet radiation. Journal of Nematology 47:184-189.

Strauch O, Oestergaard J, Hollmer S, Ehlers R-U (2004) Genetic improvement of the desiccation tolerance of the entomopathogenic nematode Heterorhabditis bacteriophora through selective breeding. Biological Control 31:218-226.

Sumaya NH, Aryal S, Vandenbossche B, Barg M, Doerfler V, Strauch O, Molina C, Ehlers R-U (2017) Phenotyping dauer juvenile oxidative stress tolerance, longevity and persistence within wild type and inbred lines of the entomopathogenic nematode Heterorhabditis bacteriophora. Nematology 19: 971-986.

Toepfer S, Gueldenzoph C, Ehlers R-U, Kuhlmann U (2005) Screening of entomopathogenic nematodes for virulence against the invasive western corn rootworm, Diabrotica virgifera virgifera (Coleoptera: Chrysomelidae) in Europe. Bulletin of Entomological Research 95:473-82.

Toepfer S, Burger R, Ehlers R-U, Peters A, Kuhlmann U (2010) Controlling Western Corn Rootworm larvae with entomopathogenic nematodes: Effect of application techniques on plant-scale efficacy. Journal of Applied Entomology 134: 467-480.

Vadnal J, Ratnappan R, Keaney M, Kenney E, Eleftherianos I, O'Halloran D, Hawdon JM (2017) Identification of candidate infection genes from the model entomopathogenic nematode Heterorhabditis bacteriophora. BMC Genomics, 18: 8.

## Claims

1. Entomopathogenic nematode *Heterorhabditis bacteriophora* having an enhanced longevity, comprising

- a first locus comprising a single nucleotide polymorphism at position 75 of the nucleotide sequence SC00004647 as depicted in SEQ ID NO: 5, in which C is substituted by T; and/or
- a second locus comprising a single nucleotide polymorphism at position 54 of the nucleotide sequence SC00006203 as depicted in SEQ ID NO: 7, in which C is substituted by T.

2. Entomopathogenic nematode according to claim 1, having

- a third locus comprising a single nucleotide polymorphism at position 66 of the nucleotide sequence SC00003427 as depicted in SEQ ID NO: 1, in which T is substituted by G; and/or
- a fourth locus comprising a single nucleotide polymorphism at position 76 of the nucleotide sequence SC00004141 as depicted in SEQ ID NO: 2, in which A is substituted by T; and/or
- a fifth locus comprising a single nucleotide polymorphism at position 86 of the nucleotide sequence SC00004634 as depicted in SEQ ID NO: 4, in which C is substituted by T; and/or
- a sixth locus comprising a single nucleotide polymorphism at position 98 of the nucleotide sequence SC00005330 as depicted in SEQ ID NO: 6, in which G is substituted by A; and/or
- a seventh locus comprising a single nucleotide polymorphism at position 77 of the nucleotide sequence SC00012917 as depicted in SEQ ID NO: 9, in which C is substituted by G; and/or
- an eighth locus comprising a single nucleotide polymorphism at position 200 of the nucleotide sequence EN-Hb_oxid-11688 as depicted in SEQ ID NO: 10, in which G is substituted by C; and/or
- a ninth locus comprising a single nucleotide polymorphism at position 176 of the nucleotide sequence EN-Hb_oxid-26008 as depicted in SEQ ID NO: 11, in which A is substituted by G.

3. Entomopathogenic nematode according to one of the preceding claims, **characterized by** tenth locus conferring an enhanced virulence, comprising a single nucleotide polymorphism at position 73 of the nucleotide sequence SC00004554 as depicted in SEQ ID NO: 3, in which G is substituted by A.

4. Entomopathogenic nematode according to one of the preceding claims, **characterized by** a eleventh locus conferring an enhanced virulence, comprising a single nucleotide polymorphism at position 111 of the nucleotide sequence SC00010093 as depicted in SEQ ID NO: 8, in which G is substituted by A.

**5.** Method of identifying at least one individual of entomopathogenic nematode *Heterorhabditis bacteriophora* associated with enhanced longevity, **characterized by** determining a first locus comprising a single nucleotide polymorphism at position 75 of the nucleotide sequence SC00004647 as depicted in SEQ ID NO: 5, in which C is substituted by T; and/or a second locus comprising a single nucleotide polymorphism at position 54 of the nucleotide sequence SC00006203 as depicted in SEQ ID NO: 7, in which C is substituted by T.

**6.** Method according to claim 4, wherein the individual **characterized by** determining

- a third locus comprising a single nucleotide polymorphism at position 66 of the nucleotide sequence SC00003427 as depicted in SEQ ID NO: 1, in which T is substituted by G; and/or
- a fourth locus comprising a single nucleotide polymorphism at position 76 of the nucleotide sequence SC00004141 as depicted in SEQ ID NO: 2, in which A is substituted by T; and/or
- a fifth locus comprising a single nucleotide polymorphism at position 86 of the nucleotide sequence SC00004634 as depicted in SEQ ID NO: 4, in which C is substituted by T; and/or
- a sixth locus comprising a single nucleotide polymorphism at position 98 of the nucleotide sequence SC00005330 as depicted in SEQ ID NO: 6, in which G is substituted by A; and/or
- a seventh locus comprising a single nucleotide polymorphism at position 77 of the nucleotide sequence SC00012917 as depicted in SEQ ID NO: 9, in which C is substituted by G; and/or
- an eighth locus comprising a single nucleotide polymorphism at position 200 of the nucleotide sequence EN-Hb_oxid-11688 as depicted in SEQ ID NO: 10, in which G is substituted by C; and/or
- a ninth locus comprising a single nucleotide polymorphism at position 176 of the nucleotide sequence EN-Hb_oxid-26008 as depicted in SEQ ID NO: 11, in which A is substituted by G.

**7.** Method of identifying at least one individual of entomopathogenic nematode *Heterorhabditis bacteriophora* associated with enhanced virulence, **characterized by**

- determining a tenth locus conferring an enhanced virulence, comprising a single nucleotide polymorphism at position 73 of the nucleotide sequence SC00004554 as depicted in SEQ ID NO: 3, in which G is substituted by A; or
- determining an eleventh locus conferring an enhanced virulence, comprising a single nucleotide polymorphism at position 111 of the nucleotide sequence SC00010093 as depicted in SEQ ID NO: 8, in which G is substituted by A.

**8.** Biological control agent comprising the entomopathogenic nematode according to one of the preceding claims.

**9.** Biological control agent according to claim 8 comprising at least one agriculturally acceptable carrier for the nematodes.

**10.** Use of the entomopathogenic nematode according to one of the claims 1 to 4 against true weevils (*Curculionidae*), scarabs (*Scarabaeidae*) or leaf beetles (*Chrysomelidae*).

**11.** Use according to claim 10 against the western corn rootworm (*Diabrotica virgifera virgifera*).

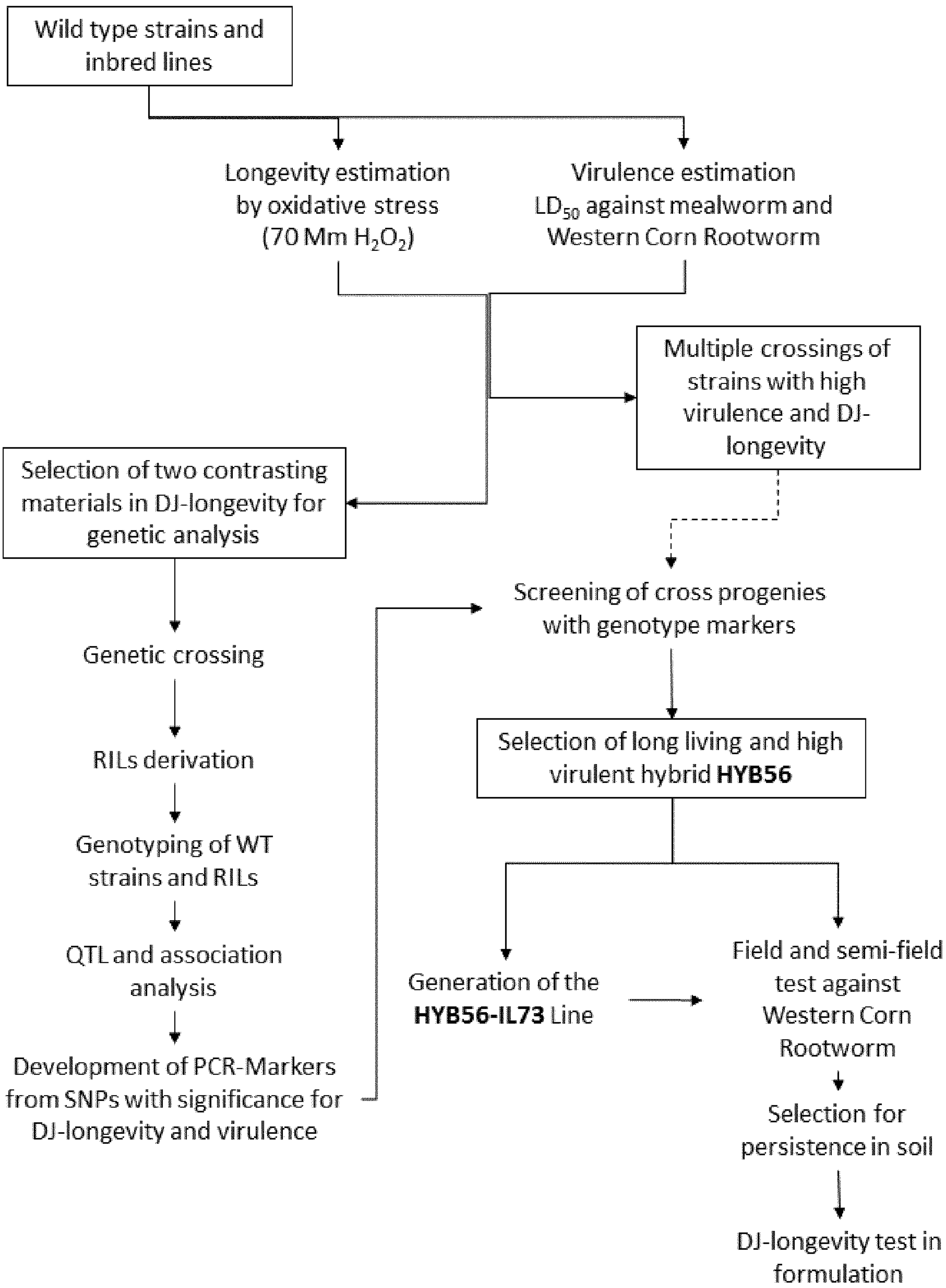

Wild type strains and inbred lines
Longevity estimation by oxidative stress (70 Mm $H_2O_2$)
Virulence estimation $LD_{50}$ against mealworm and Western Corn Rootworm
Multiple crossings of strains with high virulence and DJ-longevity
Selection of two contrasting materials in DJ-longevity for genetic analysis
Screening of cross progenies with genotype markers
Genetic crossing
Selection of long living and high virulent hybrid **HYB56**
RILs derivation
Genotyping of WT strains and RILs
QTL and association analysis
Field and semi-field test against Western Corn Rootworm
Generation of the **HYB56-IL73** Line
Development of PCR-Markers from SNPs with significance for DJ-longevity and virulence
Selection for persistence in soil
DJ-longevity test in formulation


**FIG.1**

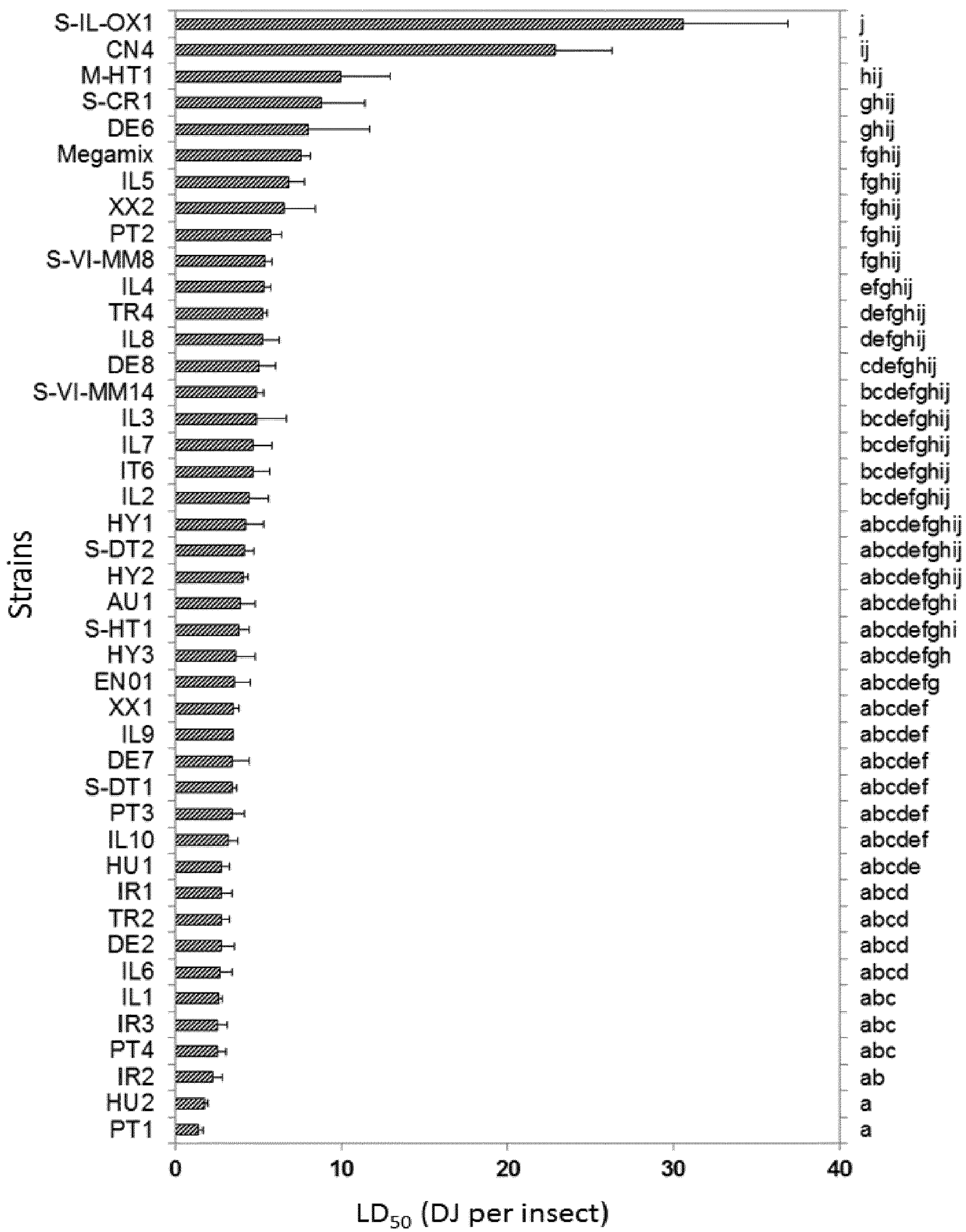
Strains
S-IL-OX1
CN4
M-HT1
S-CR1
DE6
Megamix
IL5
XX2
PT2
S-VI-MM8
IL4
TR4
IL8
DE8
S-VI-MM14
IL3
IL7
IT6
IL2
HY1
S-DT2
HY2
AU1
S-HT1
HY3
EN01
XX1
IL9
DE7
S-DT1
PT3
IL10
HU1
IR1
TR2
DE2
IL6
IL1
IR3
PT4
IR2
HU2
PT1
j
ij
hij
ghij
ghij
fghij
fghij
fghij
fghij
fghij
efghij
defghij
defghij
cdefghij
bcdefghij
bcdefghij
bcdefghij
bcdefghij
bcdefghij
abcdefghij
abcdefghij
abcdefghij
abcdefghi
abcdefghi
abcdefgh
abcdefg
abcdef
abcdef
abcdef
abcdef
abcdef
abcdef
abcde
abcd
abcd
abcd
abcd
abc
abc
abc
ab
a
a
0
10
20
30
40
LD50 (DJ per insect)

**FIG.2**

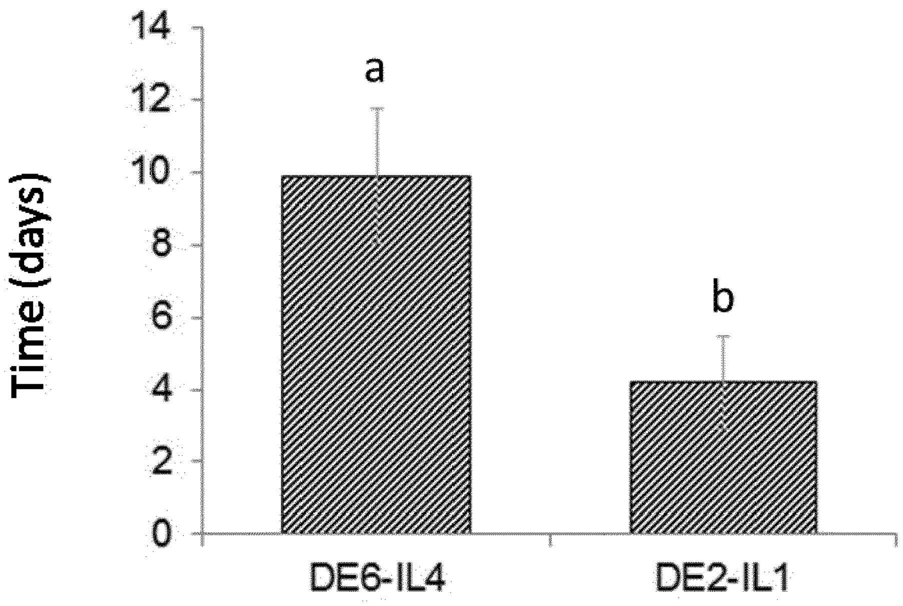


**FIG.3**

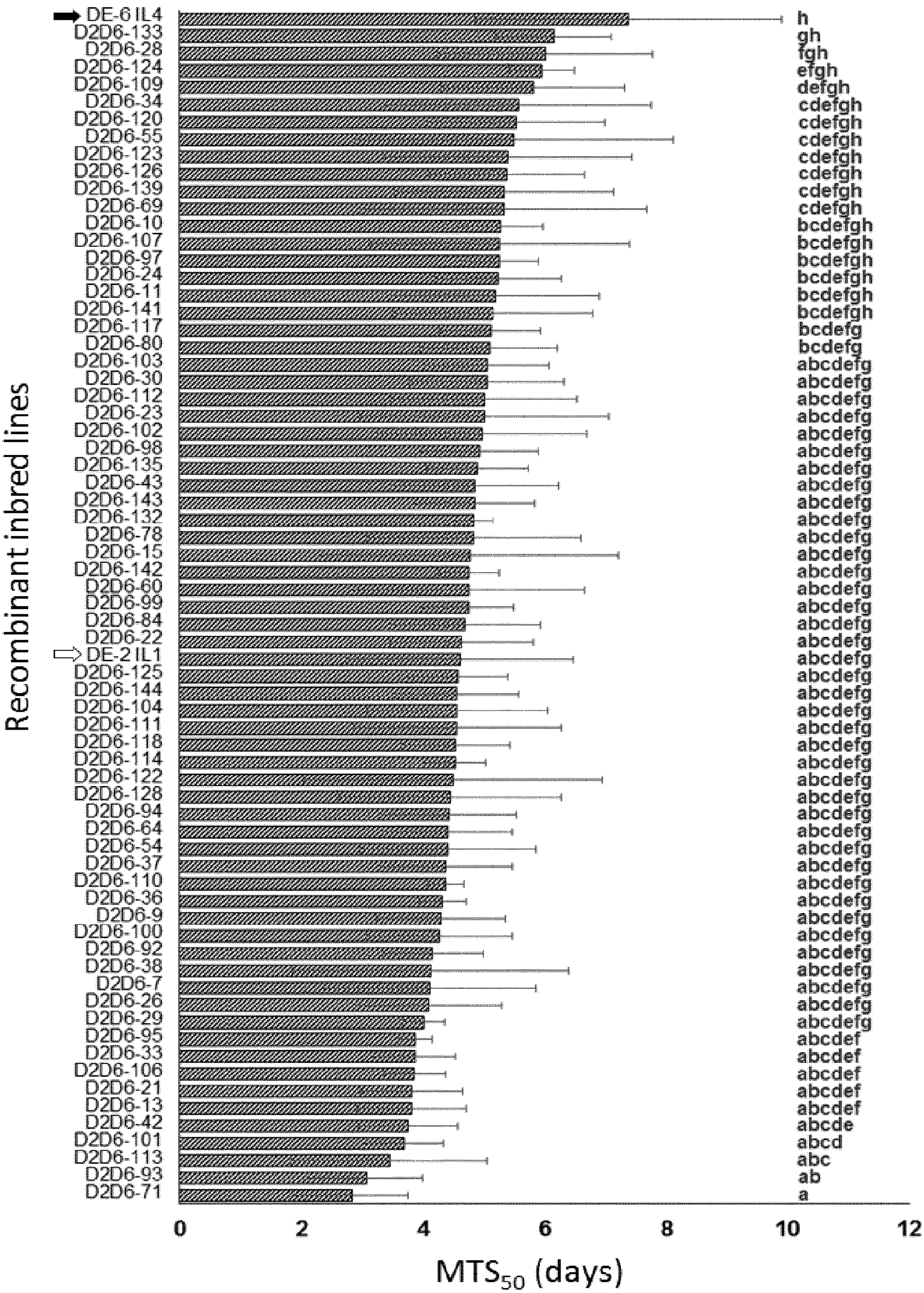
DE-6 IL4
D2D6-133
D2D6-28
D2D6-124
D2D6-109
D2D6-34
D2D6-120
D2D6-55
D2D6-123
D2D6-126
D2D6-139
D2D6-69
D2D6-10
D2D6-107
D2D6-97
D2D6-24
D2D6-11
D2D6-141
D2D6-117
D2D6-80
D2D6-103
D2D6-30
D2D6-112
D2D6-23
D2D6-102
D2D6-98
D2D6-135
D2D6-43
D2D6-143
D2D6-132
D2D6-78
D2D6-15
D2D6-142
D2D6-60
D2D6-99
D2D6-84
D2D6-22
DE-2 IL1
D2D6-125
D2D6-144
D2D6-104
D2D6-111
D2D6-118
D2D6-114
D2D6-122
D2D6-128
D2D6-94
D2D6-64
D2D6-54
D2D6-37
D2D6-110
D2D6-36
D2D6-9
D2D6-100
D2D6-92
D2D6-38
D2D6-7
D2D6-26
D2D6-29
D2D6-95
D2D6-33
D2D6-106
D2D6-21
D2D6-13
D2D6-42
D2D6-101
D2D6-113
D2D6-93
D2D6-71
Recombinant inbred lines
h
gh
fgh
efgh
defgh
cdefgh
bcdefgh
bcdefg
abcdefg
abcdef
abcde
abcd
abc
ab
a
0
2
4
6
8
10
12
MTS50 (days)

FIG.4

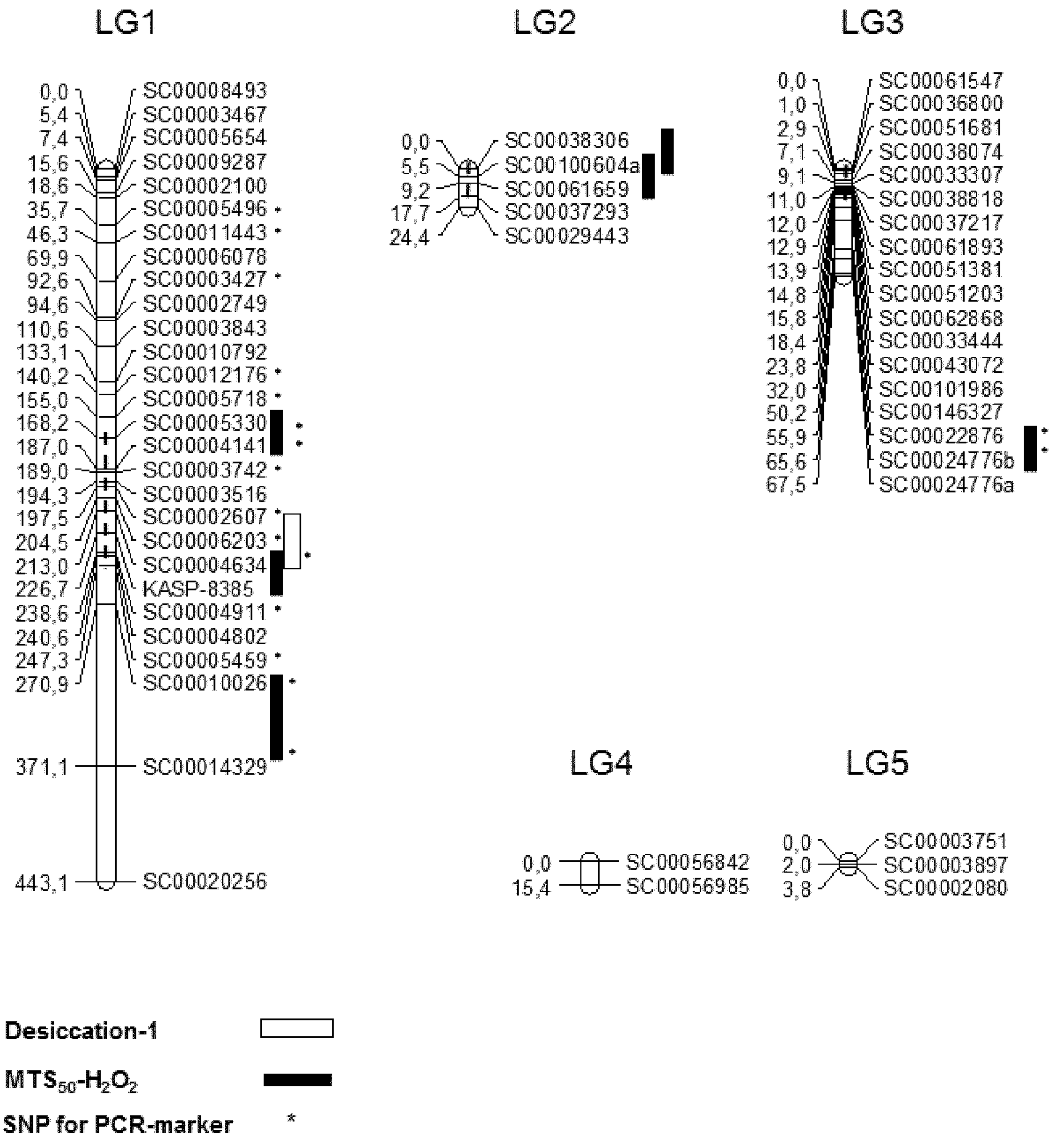
LG1
0,0 SC00008493
5,4 SC00003467
7,4 SC00005654
15,6 SC00009287
18,6 SC00002100
35,7 SC00005496 *
46,3 SC00011443 *
69,9 SC00006078
92,6 SC00003427 *
94,6 SC00002749
110,6 SC00003843
133,1 SC00010792
140,2 SC00012176 *
155,0 SC00005718 *
168,2 SC00005330 *
187,0 SC00004141 *
189,0 SC00003742 *
194,3 SC00003516
197,5 SC00002607 *
204,5 SC00006203 *
213,0 SC00004634 *
226,7 KASP-8385
238,6 SC00004911 *
240,6 SC00004802
247,3 SC00005459 *
270,9 SC00010026 *
371,1 SC00014329 *
443,1 SC00020256
LG2
0,0 SC00038306
5,5 SC00100604a
9,2 SC00061659
17,7 SC00037293
24,4 SC00029443
LG3
0,0 SC00061547
1,0 SC00036800
2,9 SC00051681
7,1 SC00038074
9,1 SC00033307
11,0 SC00038818
12,0 SC00037217
12,9 SC00061893
13,9 SC00051381
14,8 SC00051203
15,8 SC00062868
18,4 SC00033444
23,8 SC00043072
32,0 SC00101986
50,2 SC00146327
55,9 SC00022876 *
65,6 SC00024776b *
67,5 SC00024776a
LG4
0,0 SC00056842
15,4 SC00056985
LG5
0,0 SC00003751
2,0 SC00003897
3,8 SC00002080
Desiccation-1
MTS50-H2O2
SNP for PCR-marker *

FIG.5

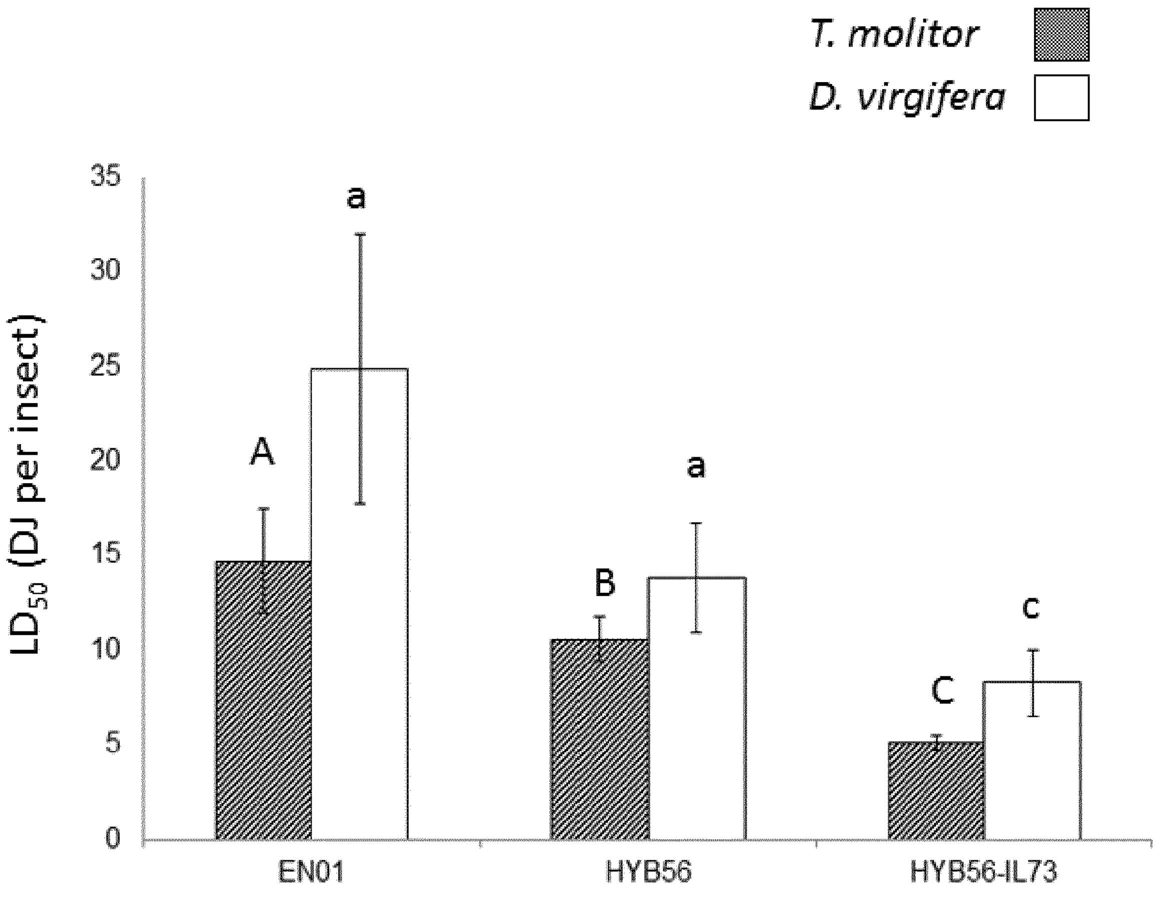
T. molitor
D. virgifera
35
30
25
20
15
10
5
0
LD50 (DJ per insect)
a
A
a
B
c
C
EN01
HYB56
HYB56-IL73

**FIG.6**

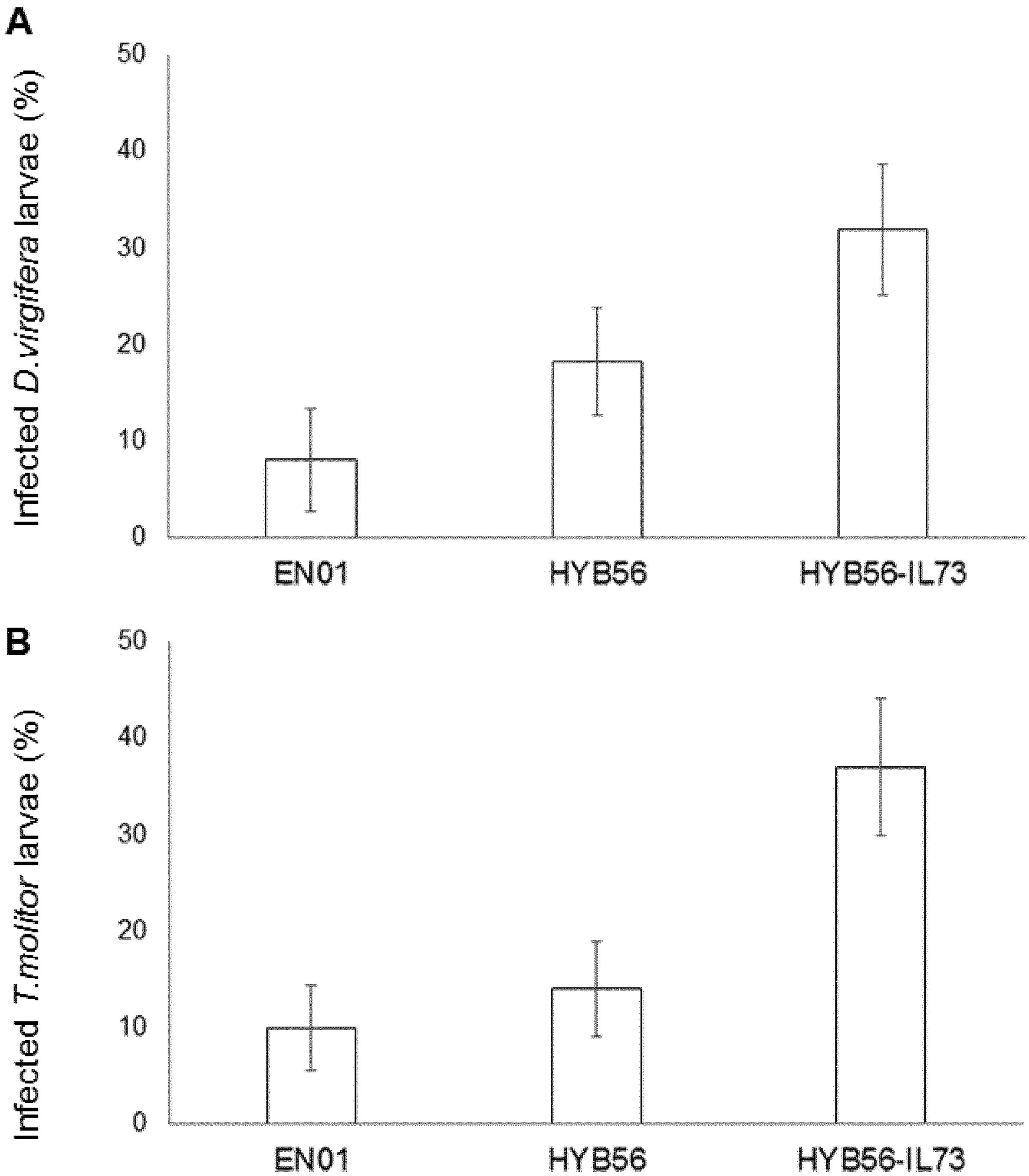
A
50
40
30
20
10
0
Infected D.virgifera larvae (%)
EN01
HYB56
HYB56-IL73
B
50
40
30
20
10
0
Infected T.molitor larvae (%)
EN01
HYB56
HYB56-IL73

FIG.7

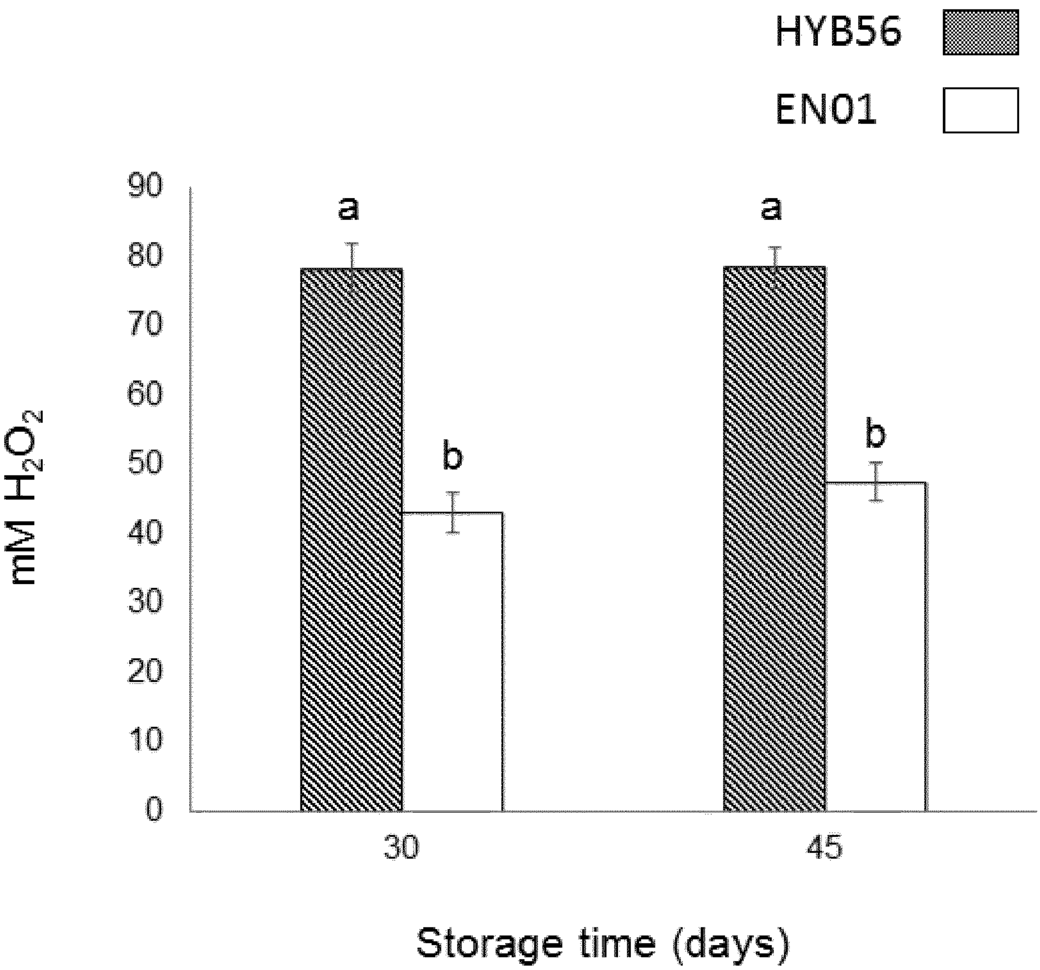
HYB56
EN01
90
80
70
60
50
40
30
20
10
0
a
a
b
b
mM $H_2O_2$
30
45
Storage time (days)

FIG.8

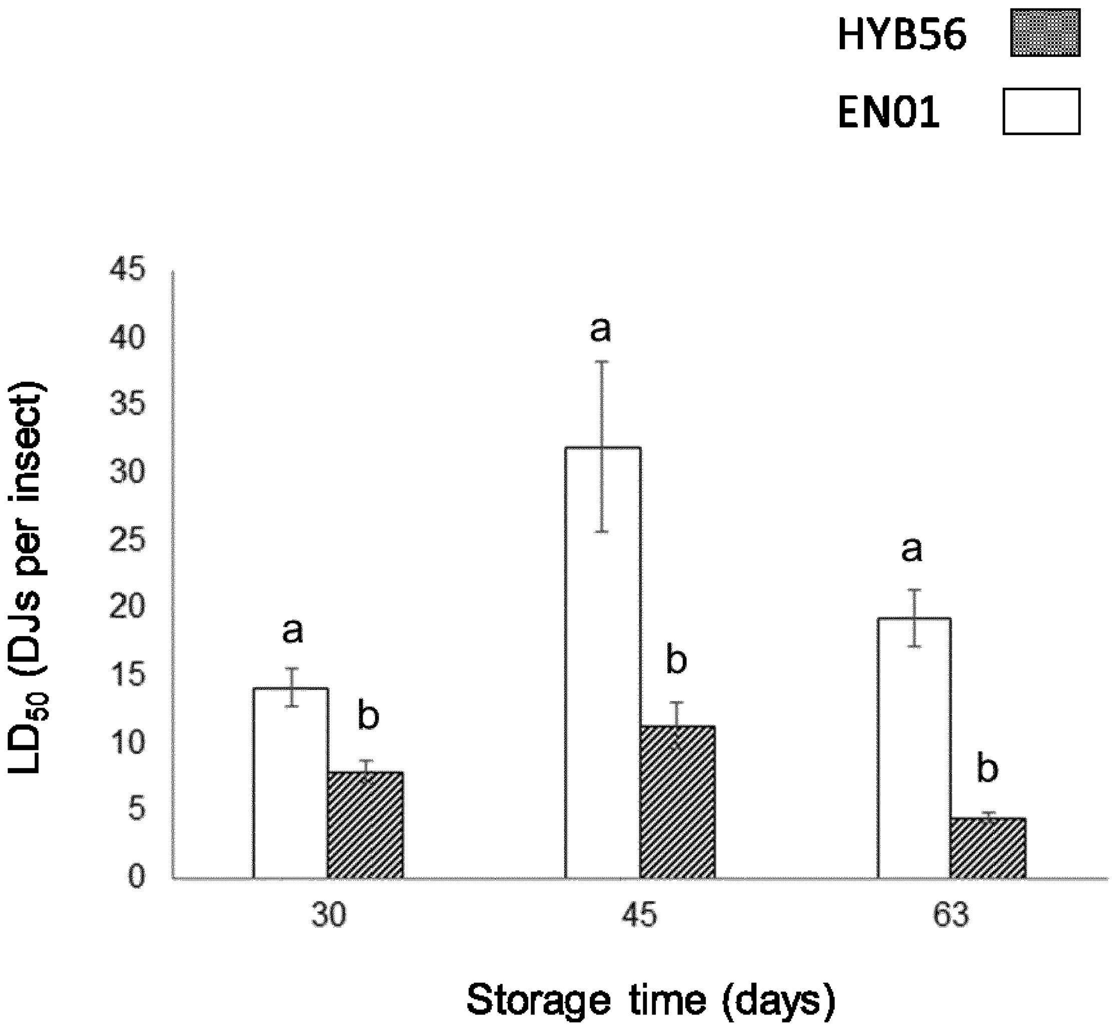
HYB56
EN01
45
40
35
30
25
20
15
10
5
0
LD50 (DJs per insect)
a
b
a
b
a
b
30
45
63
Storage time (days)

FIG.9

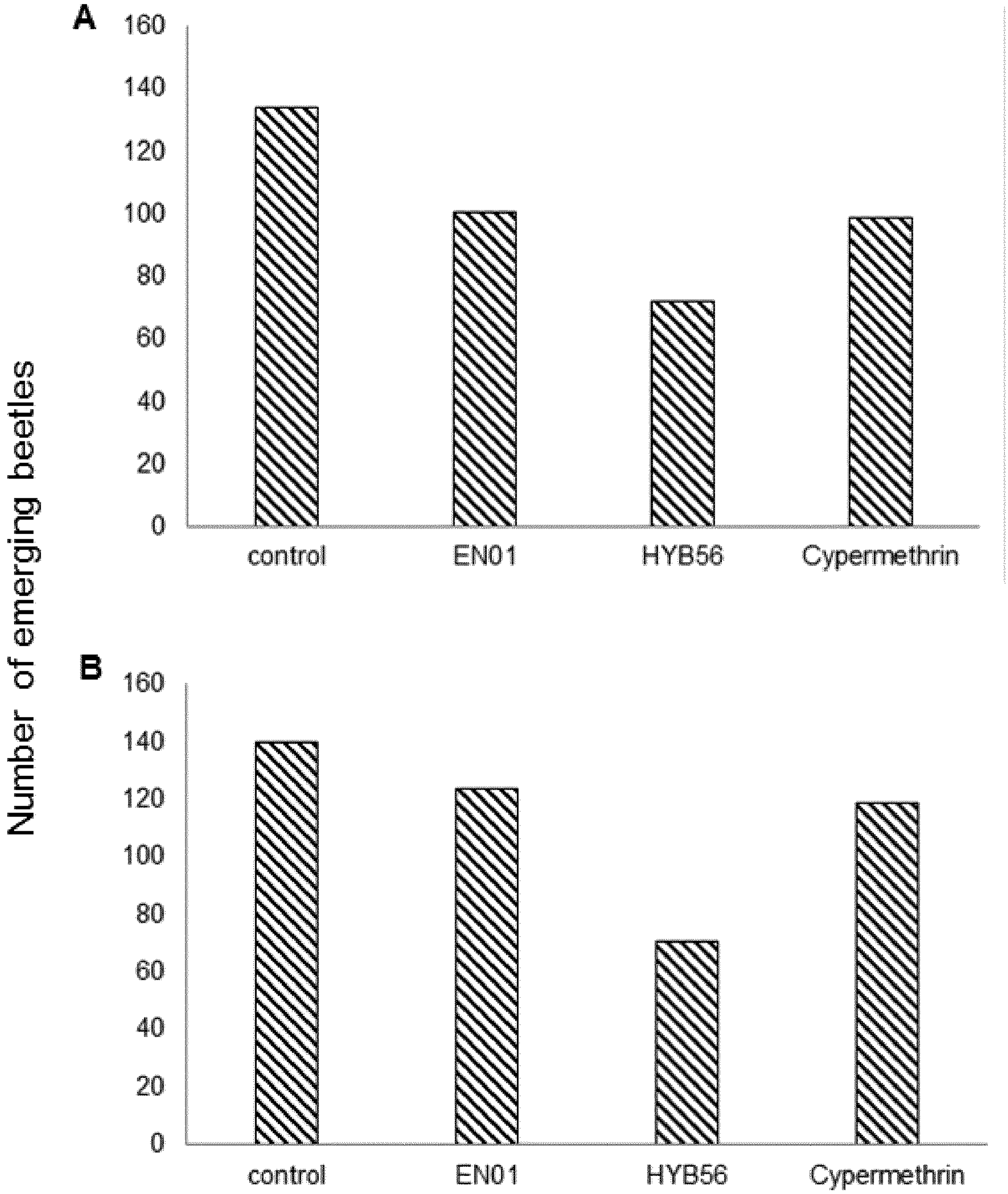
A
B
Number of emerging beetles
160
140
120
100
80
60
40
20
0
control
EN01
HYB56
Cypermethrin

FIG.10

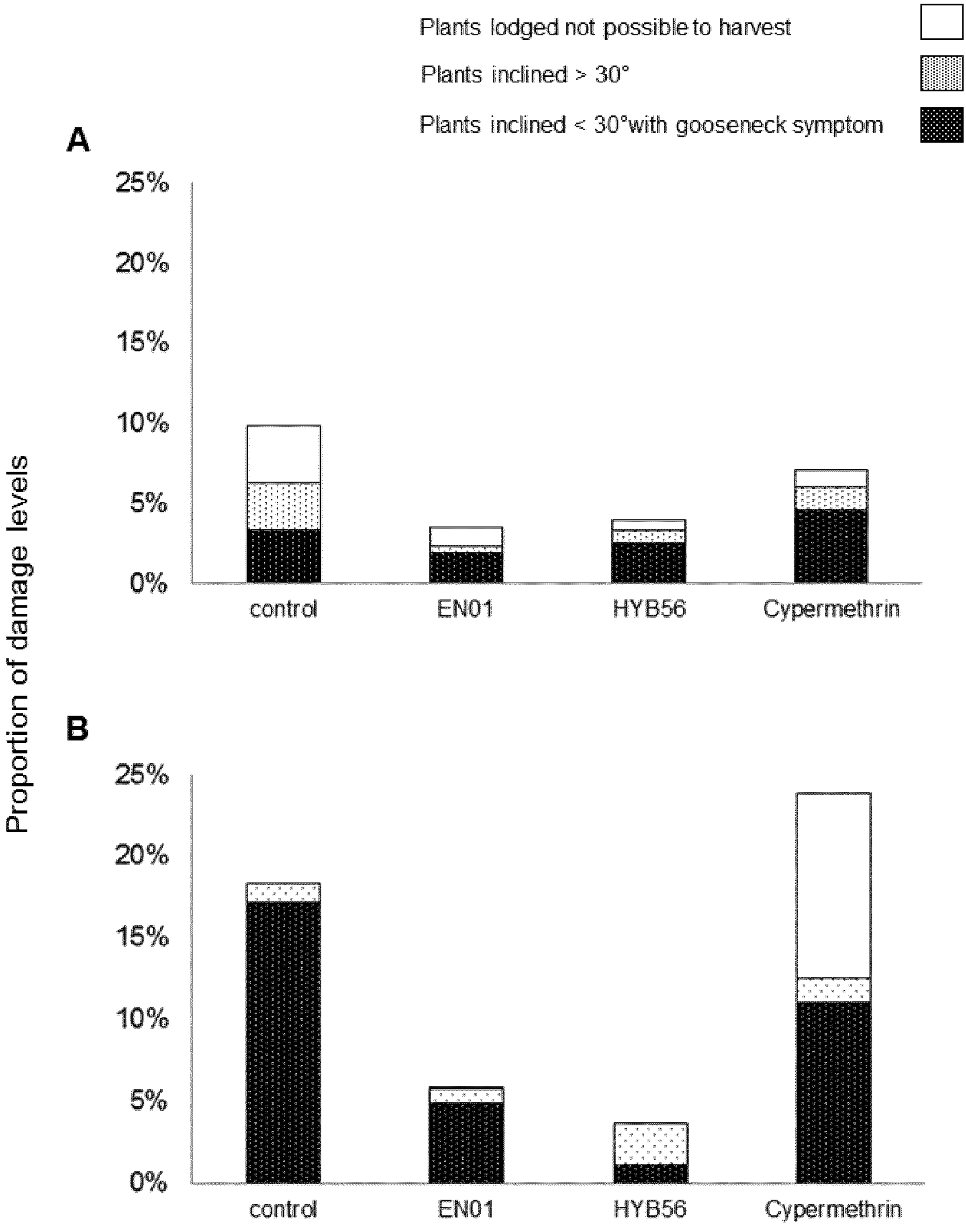
Plants lodged not possible to harvest
Plants inclined > 30°
Plants inclined < 30°with gooseneck symptom
A
B
Proportion of damage levels
25%
20%
15%
10%
5%
0%
control
EN01
HYB56
Cypermethrin

FIG.11

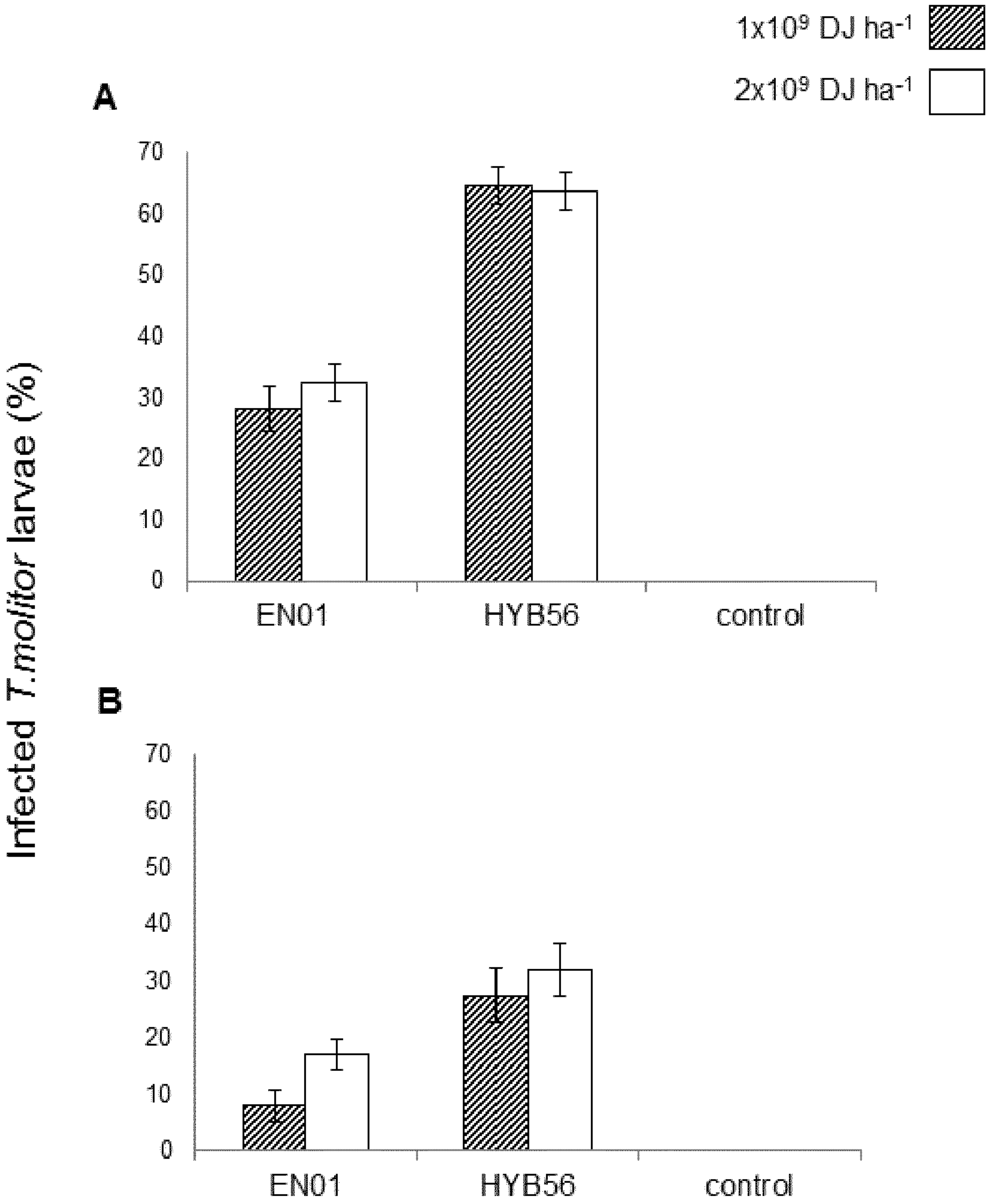
1x10^9 DJ ha^-1
2x10^9 DJ ha^-1
A
B
Infected T.molitor larvae (%)
70
60
50
40
30
20
10
0
EN01
HYB56
control

FIG.12

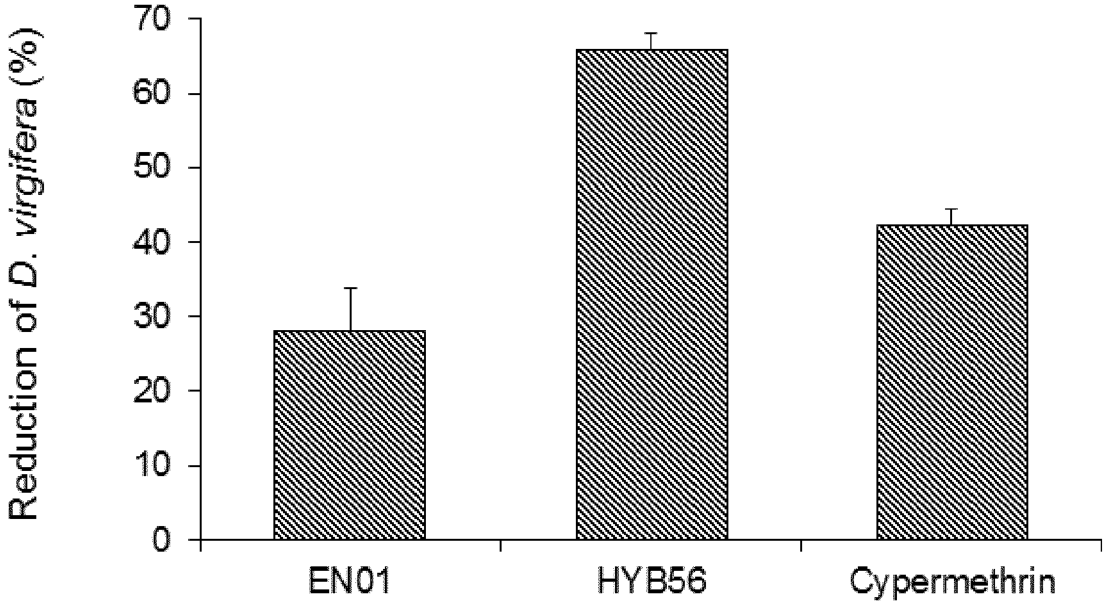
70
60
50
40
30
20
10
0
Reduction of D. virgifera (%)
EN01
HYB56
Cypermethrin

**FIG.13**

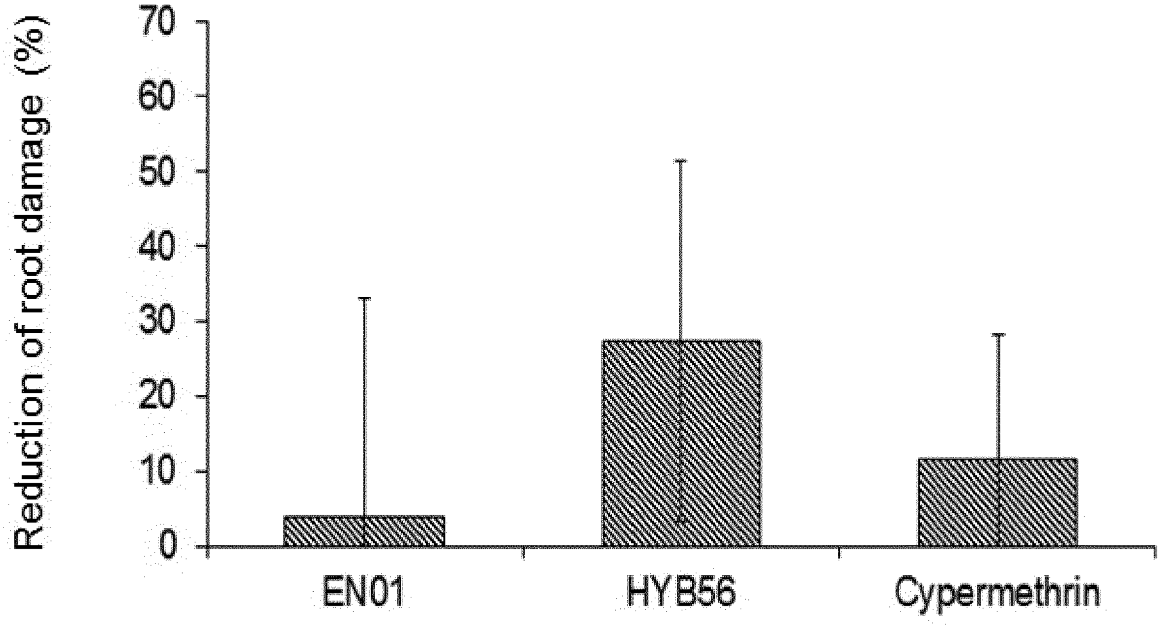
Reduction of root damage (%)
70
60
50
40
30
20
10
0
EN01
HYB56
Cypermethrin

FIG.14

Europäisches Patentamt
European Patent Office
Office européen des brevets

# EUROPEAN SEARCH REPORT

Application Number
EP 18 18 6046

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SUMAYA NANETTE HOPE ET AL: "Applying inbreeding, hybridization and mutagenesis to improve oxidative stress tolerance and longevity of the entomopathogenic nematodeHeterorhabditis bacteriophora", JOURNAL OF INVERTEBRATE PATHOLOGY, SAN DIEGO, CA, US, vol. 151, 16 November 2017 (2017-11-16), pages 50-58, XP085337901, ISSN: 0022-2011, DOI: 10.1016/J.JIP.2017.11.001 * Abstract; p.51, col.1; Fig.1; Section 2.6-2.8; Discussion * ----- | 1-11 | INV. A01N63/00 A01K67/033 |
| X | Itamar Glazer ET AL: "Overview of the research focused on the application of entomopathogenic nematodes in Europe", , 1 January 2016 (2016-01-01), XP055522443, DOI: 10.13140/RG.2.2.29477.50408 Retrieved from the Internet: URL:https://www.researchgate.net/profile/Apostolos_Kapranas/publication/312525774_Overview_of_the_research_focused_on_the_application_of_entomopathogenic_nematodes_in_Europe/links/5880a23945851503b6edd3d4/Overview-of-the-research-focused-on-the-application-of-entomopathogenic-nematodes-in-Europe.pdf * Whole poster, especially conclusions * ----- -/-- | 1-11 | TECHNICAL FIELDS SEARCHED (IPC) A01N A01K |

The present search report has been drawn up for all claims

2

| Place of search | Date of completion of the search | Examiner |
|---|---|---|
| Munich | 13 February 2019 | Roscoe, Richard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 18 18 6046

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | XIAODONG BAI ET AL: "A Lover and a Fighter: The Genome Sequence of an Entomopathogenic Nematode Heterorhabditis bacteriophora", PLOS ONE, vol. 8, no. 7, 18 July 2013 (2013-07-18), page e69618, XP055522284, DOI: 10.1371/journal.pone.0069618 * p.3-4; Fig.2 * ----- | 1-11 | |
| Y | HONDA Y ET AL: "The daf-2 gene network for longevity regulates oxidative stress resistance and Mn-superoxide dismutase gene expression in Caenorhabditis elegans", THE FASEB JOURNAL, FEDERATION OF AMERICAN SOCIETIES FOR EXPERIMENTAL BIOLOGY, US, vol. 13, no. 11, 1 August 1999 (1999-08-01), pages 1385-1393, XP002396966, ISSN: 0892-6638 * Abstract; Fig.5 * ----- | 1-11 | TECHNICAL FIELDS SEARCHED (IPC) |
| A | H. INOUE ET AL: "The DM Domain Transcription Factor MAB-3 Regulates Male Hypersensitivity to Oxidative Stress in Caenorhabditis elegans", MOLECULAR AND CELLULAR BIOLOGY., vol. 30, no. 14, 24 May 2010 (2010-05-24), pages 3453-3459, XP55555423, US ISSN: 0270-7306, DOI: 10.1128/MCB.01459-09 * Whole doc., in partic. Abstract, p.3456-3458 * ----- | 1-11 | |

The present search report has been drawn up for all claims

2

| Place of search | Date of completion of the search | Examiner |
|---|---|---|
| Munich | 13 February 2019 | Roscoe, Richard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

Application Number
EP 18 18 6046

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION SHEET B

Application Number
EP 18 18 6046

The Search Division considers that the present European patent application does not comply with the requirements of unity of invention and relates to several inventions or groups of inventions, namely:

1. claims: 1-11(partially)

Subject-matter relating to SNP C>T at position 75 in sequence SC00004647 (Seq.ID No.5), including a nematode and a method of selecting nematodes on the basis of the marker. Also combination of both said SNP and SNP at position 54.

---

2. claims: 1-11(partially)

Subject-matter relating to SNP C>T at position 54 in sequence SC00006203 (Seq.ID No.7), including a nematode and a method of selecting nematodes on the basis of the marker. Only matter where additional position 75 SNP not present ("or" aspect) .

---

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **BAI X ; ADAMS BJ ; CICHE TA ; CLIFTON S ; GAUGLER R ; HOGENHOUT SA ; SPIETH J ; STERNBERG PW ; WILSON RK ; GREWAL PS.** Transcriptomic analysis of the entomopathogenic nematode Heterorhabditis bacteriophora TTO1. *BMC Genomics,* 2009, vol. 10, 205
- **BAI X ; ADAMS BJ ; CICHE TA ; CLIFTON S ; GAUGLER R ; KIM KS ; SPIETH J ; STERNBERG PW ; WILSON RK ; GREWAL PS. et al.** A lover and a fighter: the genome sequence of an entomopathogenic nematode Heterorhabditis bacteriophora. *PLoS One,* 2013, vol. 8, e69618
- **EHLERS R-U.** Mass production of entomopathogenic nematodes for plant protection. *Applied Microbiology and Biotechnology,* 2001, vol. 56, 623-633
- **EHLERS R-U ; OESTERGAARD J ; HOLLMER S ; WINGEN M ; STRAUCH O.** Genetic selection for heat tolerance and low temperature activity of the entomopathogenic nematode bacterium-complex Heterorhabditis bacteriophora-Photorhabdus luminescens. *Biocontrol,* 2005, vol. 50, 699-716
- **ELSHIRE RJ ; GLAUBITZ JC ; SUN Q ; POLAND JA ; KAWAMOTO K ; BUCKLER ES ; MITCHELL SE.** A robust, simple genotyping-by-sequencing (GBS) approach for high diversity species. *PloS One,* 2011, vol. 6, e19379
- **GREWAL PS ; WANG X ; TAYLOR RAJ.** Dauer juvenile longevity and stress tolerance in natural populations of entomopathogenic nematodes: is there a relationship?. *International Journal for Parasitology,* 2002, vol. 32, 717-725
- Nematodes as Biocontrol Agents. CAB International Publisher, 2005
- Formulation and quality control of entomopathogenic nematodes. **GREWAL PS ; PETERS A.** Nematodes as Biocontrol Agents. CAB International Publisher, 2005, 79-90
- **GUO W ; YAN X ; ZHAO G ; HAN R.** Efficacy of entomopathogenic Steinernema and Heterorhabditis nematodes against white grubs (Coleoptera: Scarabaeidae) in peanut fields. *Journal of Economic Entomology,* 2013, vol. 106, 1112-1117
- **HILLS TM ; PETERS DC.** A method of evaluating post planting insecticide treatments for control of Western Corn Rootworm larvae. *Journal of Economic Entomology,* 1971, vol. 64, 764-765
- **IRAKI N ; SALAH N ; SANSOUR MA ; SEGAL D ; GLAZER I ; JOHNIGK SA ; HUSSEIN MA ; EHLERS R-U.** Isolation and characterization of two entomopathogenic nematode strains, Heterorhabditis indica (Nematoda, Rhabditida), from the West Bank, Palestinian Territories. *Journal of Applied Entomology,* 2000, vol. 124, 375-380
- **JOHNIGK, S-A ; EHLERS, R-U.** Endotokia matricida in hermaphrodites of Heterorhabditis spp. and the effect of the food supply. *Nematology,* 1999, vol. 1, 717-726
- Western Corn Rootworm (Diabrotica virgifera virgifera LeConte) and the crop rotation system in Europe. **KISS J ; KOMAROMI J ; BAYAR K ; EDWARDS CR ; HATALA-HSELLÉR I.** In: Vidal S, Kuhlmann U, Edwards CR (Eds) Western Corn Rootworm: Ecology and Management. CAB International Publisher, 2005, 189-220
- **KRYSAN JL ; SMITH RF.** Systematics of the virgifera species group of Diabrotica (Coleoptera: Chrysomelidae: Galerucinae). *Entomography,* 1987, vol. 5, 375-484
- **LONG SJ ; RICHARDSON PN ; FENLON JS.** Influence of temperature on the infectivity of entomopathogenic nematodes (Steinernema and Heterorhabditis spp.) to larvae and pupae of the vine weevil Otiorhynchus sulcatus (Coleoptera: Curculionidae). *Nematology,* 2000, vol. 2, 309-17
- **MUKUKA J ; STRAUCH O ; EHLERS R-U.** Variability in desiccation tolerance among different strains of the entomopathogenic nematode Heterorhabditis bacteriophora. *Nematology,* 2010, vol. 12, 711-720
- **MUKUKA J ; STRAUCH O ; NELLAS SUMAYA NH ; EHLERS R-U.** Improvement of heat and desiccation tolerance in Heterorhabditis bacteriophora through cross-breeding of tolerant strains and successive genetic selection. *BioControl,* 2010, vol. 55, 511-521
- **POINAR GO.** Description and biology of a new insect parasitic rhabditoid, Heterorhabditis bacteriophora n. Gen., n. Sp. (Rhabditida: Heterorhabditidae n. Fam.). *Nematologica,* 1975, vol. 21, 463-470

- **REGEAI S ; DOLAN K ; FITZPATRICK DA ; BROWNE J ; JONES J ; BURNELL A.** Novel primers for the amplification of nuclear DNA introns in the entomopathogenic nematode Heterorhabditis bacteriophora and their cross-amplification in seven other Heterorhabditis species. *Molecular Ecology Resources,* 2009, vol. 9, 421-424
- **SHAPIRO-ILAN DI ; HAZIR S ; LETE L.** Viability and virulence of entomopathogenic nematodes exposed to ultraviolet radiation. *Journal of Nematology,* 2015, vol. 47, 184-189
- **STRAUCH O ; OESTERGAARD J ; HOLLMER S ; EHLERS R-U.** Genetic improvement of the desiccation tolerance of the entomopathogenic nematode Heterorhabditis bacteriophora through selective breeding. *Biological Control,* 2004, vol. 31, 218-226
- **SUMAYA NH ; ARYAL S ; VANDENBOSSCHE B ; BARG M ; DOERFLER V ; STRAUCH O ; MOLINA C ; EHLERS R-U.** Phenotyping dauer juvenile oxidative stress tolerance, longevity and persistence within wild type and inbred lines of the entomopathogenic nematode Heterorhabditis bacteriophora. *Nematology,* 2017, vol. 19, 971-986
- **TOEPFER S ; GUELDENZOPH C ; EHLERS R-U ; KUHLMANN U.** Screening of entomopathogenic nematodes for virulence against the invasive western corn rootworm, Diabrotica virgifera virgifera (Coleoptera: Chrysomelidae) in Europe. *Bulletin of Entomological Research,* 2005, vol. 95, 473-82
- **TOEPFER S ; BURGER R ; EHLERS R-U ; PETERS A ; KUHLMANN U.** Controlling Western Corn Rootworm larvae with entomopathogenic nematodes: Effect of application techniques on plant-scale efficacy. *Journal of Applied Entomology,* 2010, vol. 134, 467-480
- **VADNAL J ; RATNAPPAN R ; KEANEY M ; KENNEY E ; ELEFTHERIANOS I ; O'HALLORAN D ; HAWDON JM.** Identification of candidate infection genes from the model entomopathogenic nematode Heterorhabditis bacteriophora. *BMC Genomics,* 2017, vol. 18, 8